(19) Europäisches Patentamt
European Patent Office
Office européen des brevets

(11) **EP 0 552 300 B1**

(12) **EUROPEAN PATENT SPECIFICATION**

(45) Date of publication and mention
of the grant of the patent:
**08.07.1998 Bulletin 1998/28**

(51) Int Cl.[6]: **G01R 7/10**, G01N 21/27,
G01N 21/35, G01R 23/16

(21) Application number: **91920350.5**

(22) Date of filing: **09.10.1991**

(86) International application number:
**PCT/US91/07578**

(87) International publication number:
**WO 92/07275 (30.04.1992 Gazette 1992/10)**

(54) **SPECTRAL DATA MEASUREMENT AND CORRECTION**

MESSUNG VON SPEKTRALDATEN UND KORREKTION

MESURE ET CORRECTION DE DONNEES SPECTRALES

(84) Designated Contracting States:
**DE FR GB IT NL**

(30) Priority: **12.10.1990 US 596435**
**15.10.1990 US 597910**

(43) Date of publication of application:
**28.07.1993 Bulletin 1993/30**

(73) Proprietor: **EXXON RESEARCH AND**
**ENGINEERING COMPANY**
**Florham Park, New Jersey 07932-0390 (US)**

(72) Inventor: **BROWN, James, Milton**
**Flemington, NJ 08822 (US)**

(74) Representative: **Somers, Harold Arnold et al**
**ESSO Engineering (Europe) Ltd.**
**Patents & Licences**
**Mailpoint 72**
**Esso House**
**Ermyn Way**
**Leatherhead, Surrey KT22 8XE (GB)**

(56) References cited:
EP-A- 0 299 652          EP-A- 0 415 401
US-A- 4 866 644          US-A- 4 873 655
US-A- 4 985 844          US-A- 5 005 145

- TRAC, TRENDS IN ANALYTICAL CHEMISTRY
vol. 3, no. 8 , September 1984 , CAMBRIDGE GB
pages 204 - 210 H.MARTENS ET AL. 'Multivariate
calibration. I. Concepts and distinctions'
- TRAC, TRENDS IN ANALYTICAL CHEMISTRY
vol. 3, no. 10 , November 1984 , CAMBRIDGE GB
pages 266 - 271 T.NAES ET AL. 'Multivariate
calibration. II. Chemometric methods'
- ANALYTICAL CHEMISTRY vol. 59, no. 17 , 1
September 1987 , COLUMBUS US pages 1007A
- 1017A K.R.BEEBE ET AL. 'An Introduction to
Multivariate Calibration and Analysis'

**Description**

## BACKGROUND OF THE INVENTION

This invention relates, in its broadest aspect, to correcting measured spectral data of a number of samples for the effects of data arising from the measurement process itself (rather than from the sample components). However, it finds particular application to a method of estimating unknown property and/or composition data of a sample, incorporating steps to provide correction for such measurement process spectral data. Examples of property and composition data are chemical composition measurements (such as the concentration of individual chemical components as, for example, benzene, toluene, xylene, or the concentrations of a class of compounds as, for example, paraffin), physical property measurements (such as density, index of refraction, hardness, viscosity, flash point, pour point, vapor pressure), performance property measurement (such as octane number, cetane number, combustibility), and perception (smell/odor, color).

The infrared (12500 - 400 cm$^{-1}$) spectrum of a substance contains absorption features due to the molecular vibrations of the constituent molecules. The absorptions arise from both fundamentals (single quantum transitions occurring in the mid-infrared region from 4000 - 400 cm$^{-1}$) and combination bands and overtones (multiple quanta transitions occurring in the mid- and the near-infrared region from 12500 - 4000 cm$^{-1}$). The position (frequency or wavelength) of these absorptions contain information as to the types of molecular structures that are present in the material, and the intensity of the absorptions contains information about the amounts of the molecular types that are present. To use the information in the spectra for the purpose of identifying and quantifying either components or properties requires that a calibration be performed to establish the relationship between the the absorbances and the component or property that is to be estimated. For complex mixtures, where considerable overlap between the absorptions of individual constituents occurs, such calibrations must be accomplished using multivariate data analysis methods.

In complex mixtures, each constituent generally gives rise to multiple absorption features corresponding to different vibrational motions. The intensities of these absorptions will all vary together in a linear fashion as the concentration of the constituent varies. Such features are said to have intensities which are correlated in the frequency (or wavelength) domain. This correlation allows these absorptions to be mathematically distinguished from random spectral measurement noise which shows no such correlation. The linear algebra computations which separate the correlated absorbance signals from the spectral noise form the basis for techniques such as Principal Components Regression (PCR) and Partial Least Squares (PLS). As is well known, PCR is essentially the analytical mathematical procedure of Principal Components Analysis (PCA), followed by regression analysis. Reference is directed to "An Introduction to Multivariate Calibration and Analysis", Analytical Chemistry Vol. 59, No. 17, September, 1987, pages 1007 to 1017, for an introduction to Multiple Linear Regression (MLR), PCR, and PLS

PCR and PLS have been used to estimate elemental and chemical compositions and to a lesser extent physical or thermodynamic properties of solids and liquids based on their mid- or near-infrared spectra. These methods involve: [1] the collection of mid- or near-infrared spectra of a set of representative samples; [2] mathematical treatment of the spectral data to extract the Principal Components or latent variables (e.g. the correlated absorbance signals described above); and [3] regression of these spectral variables against composition and/or property data to build a multivariate model. The analysis of new samples then involves the collection of their spectra, the decomposition of the spectra in terms of the spectral variables, and the application of the regression equation to calculate the composition/properties.

The mathematical/statistical treatment of spectral data using PCR or PLS does not differentiate among possible sources of signals which are correlated in the frequency domain. In particular, PCR and PLS do not differentiate between signals arising from variations in sample components and signals arising from variations in the spectral measurement process. For mid- and near-infrared spectra, common measurement process signals include, but are not limited to, variations in the spectral baseline due to changes in instrument performance or changes in cell window transmittance, and signals due to water vapor and/or carbon dioxide in the spectrometer light path. These measurement process signals can contribute to the Principal Components or latent variables obtained by PCR or PLS, and may be correlated to the composition/property data during the regression. The resultant regression model will then be sensitive to variations in these measurement process variables, and measured compositions or properties can be in error.

In addition to sensitivity to measurement process signals, methods based on PCR or PLS do not correct for variations in the overall scaling of the spectral data. Such scaling variations can result from a variety of factors including variations in cell pathlength due to positioning of the cell in the spectrometer, and expansion or contraction of the cell during use. For situations where the sample flows through the cell during the measurement, variations in flow can also cause variations in the scaling of the spectral data which are equivalent in effect to variations in pathlength. PCR and PLS models require that spectral data be scaled to a specified pathlength prior to analysis, thus requiring that the pathlength be separately measured. The separate measurement of the cell pathlength prior to the use of the cell in collection of the sample spectrum is not convenient or in some cases (e.g. for an on-line flow cell) not possible, nor does such separate measurement necessarily account for the sources of variation mentioned above. Errors in the measured pathlength produce proportional errors in the composition/property data estimated by PCR and PLS models.

EP-A- 0 299 652 describes a method of baseline correction of a chromatogram by orthonormal substraction of spectra representing sources of systematical error, such as absorption due to eluting solvents. Peak identification in the corrected chromatogram is performed by correlation with spectra of known compounds.

## SUMMARY OF THE INVENTION

The present invention addresses in particular the problem of how to correct the measured spectral data of the calibration samples so that the data is substantially insensitive to measurement process signals. In general, the invention also seeks to provide a generally improved method which estimates unknown property and/or compositional data of a sample under consideration but which is essentially insensitive to spectral data due to the measurement process itself. It also addresses the problem of scaling variations, referred to in the preceding paragraph.

The present invention concerns a method according to claim 1 and an apparatus, for carrying out this method, according to claim 7.

Therefore, the invention relates, in its broadest aspect, to a method of correcting spectral data of a number of samples for the effects of data arising from the measurement process itself (rather than from the sample components), but it finds particular application to estimating unknown property and/or composition data of a sample where the estimation includes steps to effect the aforesaid correction for the measurement process spectral data. The spectral data for $n$ calibration samples is quantified at $f$ discrete frequencies to produce a matrix $\mathbf{X}$ (of dimension $f$ by $n$) of calibration data. The first step in the method involves producing a correction matrix $\mathbf{U_m}$ of dimension $f$ by $m$ comprising m digitized correction spectra at the discrete frequencies $f$, the correction spectra simulating data arising from the measurement process itself. The other step involves orthoganalizing $\mathbf{X}$ with respect to $\mathbf{U_m}$ to produce a corrected spectra matrix $\mathbf{X_c}$ whose spectra are orthogonal to all the spectra in $\mathbf{U_m}$. Due to this orthogonality, the spectra in matrix $\mathbf{X_c}$ are statistically independent of spectra arising from the measurement process itself. If (as would normally be the case) the samples are calibration samples used to build a predictive model interrelating known property and composition data of the $n$ samples and their measured spectra so that the model can be used to estimate unknown property and/or composition data of a sample under consideration from its measured spectrum, the estimated property and/or composition data will be unaffected by the measurement process itself. In particular, neither baseline variations nor spectra due for example to water vapor or carbon dioxide vapor in the atmosphere of the spectrometer will introduce any error into the estimates.

It is also remarked that the spectra can be absorption spectra and the preferred embodiments described below all involve measuring absorption spectra. However, this is to be considered as exemplary and not limiting on the scope of the invention as defined by the appended claims, since the method disclosed herein can be applied to other types of spectra such as reflection spectra and scattering spectra (such as Raman scattering). Although the description given herein and with reference to the drawings relate to NIR (near-infrared) and MIR (mid-infrared), nevertheless, it will be understood that the method finds applications in other spectral measurement wavelength ranges including, for example, ultraviolet, visible spectroscopy and Nuclear Magnetic Resonance (NMR) spectroscopy.

Generally, the data arising from the measurement process itself are due to two effects. The first is due to baseline variations in the spectra. The baseline variations arise from a number of causes such as light source temperature variations during the measurement, reflectance, scattering or absorption by the cell windows, and changes in the temperature (and thus the sensitivity) of the instrument detector. These baseline variations generally exhibit spectral features which are broad (correlate over a wide frequency range). The second type of measurement process signal is due to ex-sample chemical compounds present during the measurement process, which give rise to sharper line features in the spectrum. For current applications, this type of correction generally includes absorptions due to water vapor and/or carbon dioxide in the atmosphere in the spectrometer. Absorptions due to hydroxyl groups in optical fibers could also be treated in this fashion. Corrections for contaminants present in the samples can also be made, but generally only in cases where the concentration of the contaminant is sufficiently low as to not significantly dilute the concentrations of the sample components, and where no significant interactions between the contaminant and sample component occurs. It is important to recognize that these corrections are for signals that are not due to components in the sample. In this context, "sample" refers to that material upon which property and/or component concentration measurements are conducted for the purpose of providing data for the model development. By "contaminant," we refer to any material which is physically added to the sample after the property/component measurement but before or during the spectral measurement.

The present inventive method can be applied to correct only for the effect of baseline variations, in which case these variations can be modeled by a set of preferably orthogonal, frequency (or wavelength) dependent polynomials which form the matrix $\mathbf{U_m}$ of dimension $f$ by $m$ where $m$ is the order of the polynomials and the columns of $\mathbf{U_m}$ are preferably orthogonal polynomials, such as Legendre polynomials. Alternatively the inventive method can be applied to correct only for the effect of ex-sample chemical compounds (e.g. due to the presence in the atmosphere of carbon dioxide and/or water vapor). In this case, the spectra that form the columns of $U_m$ are preferably orthogonal vectors that are representative of the spectral interferences produced by such chemical compounds. It is preferred, however, that both baseline variations and ex-sample chemical compounds are modeled in the manner described to form two correction matrices $U_p$ of dimension $f$ by $p$ and $X_s$, respectively. These matrices are then combined into the single matrix $U_m$, whose columns are the columns of $U_p$ and $X_s$ arranged side-by-side.

In a preferred way of performing the invention, in addition to matrix $X$ of spectral data being orthogonalized relative to the correction matrix $U_m$, the spectra or columns of $U_m$ are all mutually orthogonal. The production of the matrix $U_m$ having mutually orthogonal spectra or columns can be achieved by firstly modeling the baseline variations by a set of orthogonal frequency (or wavelength) dependent polynomials which are computer generated simulations of the baseline variations and form the matrix $U_p$, and then at least one, and usually a plurality, of spectra of ex-sample chemical compounds (e.g. carbon dioxide and water vapor) which are actual spectra collected on the instrument, are supplied to form the matrix $X_s$. Next the columns of $X_s$ are orthogonalized with respect to $U_p$ to form a new matrix $X_s'$. This removes baseline effects from ex-sample chemical compound corrections. Then, the columns of $X_s'$ are orthogonalized with respect to one another to form a new matrix $U_s$, and lastly $U_p$ and $U_s$ are combined to form the correction matrix $U_m$, whose columns are the columns of $U_p$ and $U_s$ arranged side-by-side. It would be possible to change the order of the steps such that firstly the columns of $X_s$ are orthogonalized to form a new matrix of vectors and then the (mutually orthogonal) polynomials forming the matrix $U_p$ are orthogonalized relative to these vectors and then combined with them to form the correction matrix $U_m$. However, this is less preferred because it defeats the advantage of generating the polynomials as being orthogonal in the first place, and it will also mix the baseline variations in with the spectral variations due to ex-sample chemical compounds and make them less useful as diagnostics of instrument performance.

In a real situation, the sample spectral data in the matrix $X$ will include not only spectral data due to the measurement process itself but also data due to noise. Therefore, once the matrix $X$ (dimension $f$ by $n$) has been orthogonalized with respect to the correction matrix $U_m$ (dimension $f$ by $m$), the resulting corrected spectral matrix $X_c$ will still contain noise data. This can be removed in the following way. Firstly, a singular value decomposition is performed on matrix $X_c$ in the form $X_c = U\Sigma V^t$, where $U$ is a matrix of dimension $f$ by $n$ and contains the principal component spectra as columns, $\Sigma$ is a diagonal matrix of dimension $n$ by $n$ and contains the singular values, and $V$ is a matrix of dimension $n$ by $n$ and contains the principal component scores, $V^t$ being the transpose of $V$. In general, the principal components that correspond to noise in the spectral measurements in the original $n$ samples will have singular values which are small in magnitude relative to those due to the wanted spectral data, and can therefore be distinguished from the principal components due to real sample components. Accordingly, the next step in the method involves removing from $U$, $\Sigma$ and $V$ the $k+1$ through $n$ principal components that correspond to the noise, to form the new matrices $U'$, $\Sigma'$ and $V'$ of dimensions $f$ by $k$, $k$ by $k$ and $n$ by $k$, respectively. When these matrices are multiplied together, the resulting matrix, corresponding with the earlier corrected spectra matrix $X_c$, is free of spectral data due to noise.

For the selection of the number ($k$) of principal components to keep in the model, a variety of statistical tests suggested in the literature could be used but the following steps have been found to give the best results. Generally, the spectral noise level is known from experience with the instrument. From a visual inspection of the eigenspectra (the columns of matrix $U$ resulting from the singular value decomposition), a trained spectroscopist can generally recognize when the signal levels in the eigenspectra are comparable with the noise level. By visual inspection of the eigenspectra, an approximate number of terms, $k$, to retain can be selected. Models can then be built with, for example, $k$-2, $k$-1, $k$, $k$+1, $k$+2 terms in them and the standard errors and PRESS (Predictive Residual Error Sum of Squares) values are inspected. The smallest number of terms needed to obtain the desired precision in the model or the number of terms that give the minimum PRESS value is then selected. This choice is made by the spectroscopist, and is not automated. A Predicted Residual Error Sum of Squares is calculated by applying a predictive model for the estimation of property and/or component values for a test set of samples which were not used in the calibration but for which the true value of the property or component concentration is known. The difference between the estimated and true values is squared, and summed for all the samples in the test set (the square root of the quotient of the sum of squares and the number of test samples is sometimes calculated to express the PRESS value on a per sample basis). A PRESS value can be calculated using a cross validation procedure in which one or more of the calibration samples are left out of the data matrix during the calibration, and then analyzed with the resultant model, and the procedure is repeated until each sample has been left out once.

The polynomials that are used to model background variations are merely one type of correction spectrum. The difference between the polynomials and the other "correction spectra" modeling ex-sample chemical compounds is twofold. First, the polynomials may conveniently be computer-generated simulations of the background (although this is not essential and they could instead be simple mathematical expressions or even actual spectra of background variations) and can be generated by the computer to be orthogonal. The polynomials may be Legendre polynomials which are used in the actual implementation of the correction method since they save computation time. There is a well-known recursive algorithm to generate the Legendre polynomials (see, for example, G. Arfken, **Mathematical Methods for Physicists**, Academic Press, New York, N.Y., 1971, Chapter 12). Generally, each row of the $U_p$ matrix corresponds to a given frequency (or wavelength) in the spectrum. The columns of the $U_p$ matrix will be related to this frequency. The elements of the first column would be a constant, the elements of the second column would depend linearly on the frequency, the elements of the third column would depend on the square of the frequency, etc. The exact relationship is somewhat more complicated than that if the columns are to be orthogonal. The Legendre polynomials are generated to be orthonormal, so that it is not necessary to effect a singular value decomposition or a Gram-Schmidt orthogonalization to make them orthogonal. Alternatively, any set of suitable polynomial terms could be used, which are then orthogonalized using singular value decomposition or a Gram-Schmidt orthogonalization. Alternatively, actual spectra collected on the instrument to simulate background variation can be used and orthogonalized via one of these procedures. The other "correction spectra" are usually actual spectra collected on the instrument to simulate interferences due to ex-sample chemical compounds, e.g. the spectrum of water vapor, the spectrum of carbon dioxide vapor, or the spectrum of the optical fiber of the instrument. Computer generated spectra could be used here if the spectra of water vapor, carbon dioxide, etc. can be simulated. The other difference for the implementation of the correction method is that these "correction spectra" are not orthogonal initially, and therefore it is preferred that they be orthogonalized as part of the procedure. The polynomials and the ex-sample chemical compound "correction spectra" could be combined into one matrix, and orthogonalized in one step to produce the correction vectors. In practice, however, this is not the best procedure, since the results would be sensitive to the scaling of the polynomials relative to the ex-sample chemical compound "correction spectra". If the ex-sample chemical compound "correction spectra" are collected spectra, they will include some noise. If the scaling on the polynomials is too small, the contribution of the noise in these "correction spectra" to the total variance in the correction matrix $U_m$ would be larger than that of the polynomials, and noise vectors would end up being included in the ex-sample chemical compound correction vectors. To avoid this, preferably the polynomials are generated first, the ex-sample chemical compound "correction spectra" are orthogonalized to the polynomials, and then the correction vectors are generated by performing a singular value decomposition (described below) on the orthogonalized "correction spectra".

As indicated above, a preferred way of performing the correction for measurement process spectral data is firstly to generate the orthogonal set of polynomials which model background variations, then to orthoganalize any "correction spectra" due to ex-sample chemical compounds (e.g. carbon dioxide and/or water vapor) to this set to produce a set of "correction vectors", and finally to orthogonalize the resultant "correction vectors" among themselves using singular value decomposition. If multiple examples of "correction spectra", e.g. several spectra of water vapor, are used, the final number of "correction vectors" will be less than the number of initial "correction spectra". The ones eliminated correspond with the measurement noise. Essentially, principal components analysis (PCA) is being performed on the orthogonalized "correction spectra" to separate the real measurement process data being modeled from the random measurement noise.

It is remarked that the columns of the correction matrix $U_m$ do not have to be mutually orthogonal for the correction method to work, as long as the columns of the data matrix $X$ are orthogonalized to those of the correction matrix $U_m$. However, the steps for generating the $U_m$ matrix to have orthogonal columns is performed to simplify the computations required in the orthogonalization of the spectral data $X$ of the samples relative to the correction matrix $U_m$, and to provide a set of statistically independent correction terms that can be used to monitor the measurement process. By initially orthogonalizing the correction spectra $X_s$ due to ex-sample chemical compounds to $U_p$ which models background variations, any background contribution to the resulting correction spectra is removed prior to the orthogonalization of these correction spectra among themselves. This procedure effectively achieves a separation of the effects of background variations from those of ex-sample chemical compound variations, allowing these corrections to be used as quality control features in monitoring the performance of an instrument during the measurement of spectra of unknown materials, as will be discussed hereinbelow.

When applying the technique for correcting for the effects of measurement process spectral data in the development of a method of estimating unknown property and/or composition data of a sample under consideration, the following steps are performed. Firstly, respective spectra of $n$ calibration samples are collected, the spectra being quantified at $f$ discrete frequencies (or wavelengths) and forming a matrix $X$ of dimension $f$ by $n$. Then, in the manner described above, a correction matrix $U_m$ of dimension $f$ by $m$ is produced. This matrix comprises $m$ digitized correction spectra at the discrete frequencies $f$, the correction spectra simulating data arising from the measurement process itself. The next step is to orthogonalize $X$ with respect to $U_m$ to produce a corrected spectra matrix $X_c$ whose spectra are each orthogonal to all the spectra in $U_m$. The method further requires that $c$ property and/or composition data are collected for each of the $n$ calibration samples to form a matrix $Y$ of dimension $n$ by $c$ ($c \geq 1$). Then, a predictive model is determined correlating the elements of matrix $Y$ to matrix $X_c$. Different predictive models can be used, as will be explained below. The property and/or composition estimating method further requires measuring the spectrum of the sample under consideration at the $f$ discrete frequencies to form a matrix of dimension $f$ by 1. The unknown property and/or composition data of the samples is then estimated from its measured spectrum using the predictive model. Generally, each property and/or component is treated separately for building models and produces a separate $f$ by 1 prediction vector. The prediction is just the dot product of the unknown spectrum and the prediction vector. By combining all the prediction vectors into a matrix $\mathbf{P}$ of dimension $f$ by $c$, the prediction involves multiplying the spectrum matrix (a vector of dimension $f$ can be considered as a 1 by $f$ matrix) by the prediction matrix to produce a 1 by c vector of predictions for the $c$ properties and components.

As mentioned in the preceding paragraph, various forms of predictive model are possible. The predictive model can be determined from a mathematical solution to the equation $\mathbf{Y} = \mathbf{X}_c^t \mathbf{P} + \mathbf{E}$, where $\mathbf{X}_c^t$ is the transpose of the corrected spectra matrix $\mathbf{X}_c$, $\mathbf{P}$ is the predictive matrix of dimension $f$ by $c$, and $\mathbf{E}$ is a matrix of residual errors from the model and is of dimension $n$ by $c$. The validity of the equation $\mathbf{Y} = \mathbf{X}_c^t \mathbf{P} + \mathbf{E}$ follows from the inverse statement of Beer's law, which itself can be expressed in the form that the radiation-absorbance of a sample is proportional to the optical pathlength through the sample and the concentration of the radiation-absorbing species in that sample. Then, for determining the vector $\mathbf{y}_u$ of dimension 1 by $c$ containing the estimates of the $c$ property and/or composition data for the sample under consideration, the spectrum $\mathbf{x}_u$ of the sample under consideration, $\mathbf{x}_u$ being of dimension $f$ by 1, is measured and $\mathbf{y}_u$ is determined from the relationship $\mathbf{y}_u = \mathbf{x}_u^t \mathbf{P}$, $\mathbf{x}_u^t$ being the transpose of matrix $\mathbf{x}_u$.

Although, in a preferred implementation of this invention, the equation $\mathbf{Y} = \mathbf{X}_c^t \mathbf{P} + \mathbf{E}$ is solved to determine the predictive model, the invention could also be used in developing models where the equation is represented (by essentially the statement of Beer's law) as $\mathbf{X}_c = \mathbf{A}\mathbf{Y}^t + \mathbf{E}$, where $\mathbf{A}$ is an $f$ by $c$ matrix. In this case, the matrix $\mathbf{A}$ would first be estimated as $\mathbf{A} = \mathbf{X}_c \mathbf{Y}(\mathbf{Y}^t\mathbf{Y})^{-1}$. The estimation of the vector $\mathbf{y}_u$ of dimension 1 by $c$ containing the $c$ property and/or composition data for the sample under consideration from the spectrum $\mathbf{x}_u$ of the sample under consideration would then involve using the relationship $\mathbf{y}_u = \mathbf{x}_u\mathbf{A}(\mathbf{A}^t\mathbf{A})^{-1}$. This calculation, which is a constrained form of the K-matrix method, is more restricted in application, since the required inversion of $\mathbf{Y}^t\mathbf{Y}$ requires that $\mathbf{Y}$ contain concentration values for all sample components, and not contain property data.

The mathematical solution to the equation $\mathbf{Y} = \mathbf{X}_c^t \mathbf{P} + \mathbf{E}$ (or $\mathbf{X}_c = \mathbf{A}\mathbf{Y}^t + \mathbf{E}$) can be obtained by any one of a number of mathematical techniques which are known per se, such as linear least squares regression, sometimes otherwise known as multiple linear regression (MLR), principal components analysis/regression (PCA/PCR) and partial least squares (PLS). As mentioned above, an introduction to these mathematical techniques is given in "An Introduction to Multivariate Calibration and Analysis", Analytical Chemistry, Vol. 59, No. 17, September 1, 1987, Pages 1007 to 1017.

The purpose of generating correction matrix $\mathbf{U}_m$ and in orthogonalizing the spectral data matrix $\mathbf{X}$ to $\mathbf{U}_m$ is twofold: Firstly, predictive models based on the resultant corrected data matrix $\mathbf{X}_c$ are insensitive to the effects of background variations and ex-sample chemical components modeled in $\mathbf{U}_m$, as explained above. Secondly, the dot (scalar) products generated between the columns of $\mathbf{U}_m$ and those of $\mathbf{X}$ contain information about the magnitude of the background and ex-sample chemical component interferences that are present in the calibration spectra, and as such, provide a measure of the range of values for the magnitude of these interferences that were present during the collection of the calibration spectral data. During the analysis of a spectrum of a material having unknown properties and/or composition, similar dot products can be formed between the unknown spectrum, $\mathbf{x}_u$, and the columns of $\mathbf{U}_m$, and these values can be compared with those obtained during the calibration as a means of checking that the measurement process has not changed significantly between the time the calibration is accomplished and the time the productive model is applied for the estimation of properties and components for the sample under test. These dot products thus provide a means of performing a quality control assessment on the measurement process.

The dot products of the columns of $\mathbf{U}_m$ with those of the spectral data matrix $\mathbf{X}$ contain information about the degree to which the measurement process data contribute to the individual calibration spectra. This information is generally mixed with information about the calibration sample components. For example, the dot product of a constant vector (a first order polynomial) with a spectrum, will contain information about the total spectral integral, which is the sum of the integral of the sample absorptions, and the integral of the background. The information about calibration sample components is, however, also contained in the eigenspectra produced by the singular value decomposition of $\mathbf{X}_c$. It is therefore possible to remove that portion of the information which is correlated to the sample components from the dot products so as to recover values that are uncorrelated to the sample components, i.e. values that represent the true magnitude of the contributions of the measurement process signals to the calibration spectra. This is accomplished by the following steps:

(1) A matrix $\mathbf{V}_m$ of dimension $n$ by $m$ is formed as the product of $\mathbf{X}^t\mathbf{U}_m$, the individual elements of $\mathbf{V}_m$ being the dot products of the columns of $\mathbf{X}$ with those of $\mathbf{U}_m$;

(2) The corrected data matrix $\mathbf{X}_c$ is formed, and its singular value decomposition is computed as $\mathbf{U}\Sigma\mathbf{V}^t$;

(3) A regression of the form $\mathbf{V}_m = \mathbf{V}\mathbf{Z} + \mathbf{R}$ is calculated to establish the correlation between the dot products and the scores of the principal components: $\mathbf{V}\mathbf{Z}$ represents the portion of the dot products which is correlated to the sample components and the regression residuals $\mathbf{R}$ represent the portion of the dot products that are uncorrelated to the sample components, which are in fact the measurement process signals for the calibration samples;

(4) In the analysis of a sample under test, the dot products of the unknown spectrum with each of the correction spectra (columns of $\mathbf{U}_m$) are calculated to form a vector $\mathbf{v}_m$, the corrected spectrum $\mathbf{x}_c$ is calculated, the scores for the corrected spectrum are calculated as $\mathbf{v} = \mathbf{x}_c^t \mathbf{U}\Sigma^{-1}$, and the uncorrelated measurement process signal values are calculated as $r = v_m - vZ$. The magnitude of these values is then compared to the range of values in $R$ as a means of comparing the measurement process during the analysis of the unknown to that during the calibration.

It will be appreciated that the performance of the above disclosed correction method and method of estimating the unknown property and/or composition data of the sample under consideration involves extensive mathematical computations to be performed. In practice, such computations would be made by computer means comprising a computer or computers, which would be connected to the instrument in a measurement mode so as to receive the measured output spectrum of the calibration sample, ex-sample chemical compound or test sample. In a correction mode in conjunction with the operator, the computer means stores the calibration spectra to form the matrix $X$, calculates the correction matrix $U_m$, and orthogonalizes $X$ with respect to the correction matrix $U_m$. In addition, the computers means operates in a storing mode to store the $c$ known property and/or composition data for the $n$ calibration samples to form the matrix $Y$ of dimension $n$ by $c$ ($c \geq 1$). In a model building mode, the computer means determines, in conjunction with the operator, a predictive model correlating the elements of matrix $Y$ to those of matrix $X_c$. Lastly, the computer means is arranged to operate in a prediction mode in which it estimates the unknown property and/or compositional data of the sample under consideration from its measured spectrum using the determined predictive model correlating the elements of matrix $Y$ to those of matrix $X_c$.

In more detail, the steps involved according to a preferred way of making a prediction of property and/or composition data of a sample under consideration can be set out as follows. Firstly, a selection of samples for the calibrating is made by the operator or a laboratory technician. Then, in either order, the spectra and properties/composition of these samples need to be measured, collected and stored in the computer means by the operator and/or laboratory technician, together with spectra of ex-sample chemical compounds to be used as corrections. In addition, the operator selects the computer-generated polynomial correction used to model baseline variations. The computer means generates the correction matrix $U_m$ and then orthogonalizes the calibration sample spectra (matrix $X$) to produce the corrected spectra matrix $X_c$ and, if PCR is used, performs the singular value decomposition on matrix $X_c$. The operator has to select (in PCR) how many of the principal components to retain as correlated data and how many to discard as representative of (uncorrelated) noise. Alternatively, if the PLS technique is employed, the operator has to select the number of latent variables to use. If MLR is used to determine the correlation between the corrected spectra matrix $X_c$ and the measured property and/or composition data $Y$, then a selection of frequencies needs to be made such that the number of frequencies at which the measured spectra are quantized is less than the number of calibration samples. Whichever technique is used to determine the correlation (i.e. the predictive model) interrelating $X_c$ and $Y$, having completed the calibration, the laboratory technician measures the spectrum of the sample under consideration which is used by the computer means to compute predicted property and/or composition data based on the predictive model.

These and other features and advantages of the invention will now be described, by way of example, with reference to the drawings.

## BRIEF DESCRIPTION OF THE DRAWINGS

Figures 1 to 5 are scattergrams of different property and composition data when determined by conventional techniques plotted against the same data determined by the spectroscopic method disclosed herein;

Figures 6 to 10 show various graphical depictions and spectra relating to binary blends of isooctane and heptane;

Figures 11 to 17 show various graphical depictions and eigenspectra relating to the analysis of a 5-component additive package and to a comparison with the K Matrix Method;

Figures 18 to 21 relate to an example demonstrating how constrained spectral analysis can be used to remove measurement process signals in a real application.

Figures 22 and 23 illustrate the effect of correction for water vapor and chloroform, respectively, for isooctane/ heptane spectra and for the additive package of Example 9, respectively.

An algorithm, termed The Constrained Principal Spectra Analysis (CPSA), is described hereinbelow, and it's application to multivariate, spectral analysis is demonstrated. CPSA is a novel modification of Principal Components Analysis (PCA) which allows the spectroscopist to input his knowledge of the spectral measurement process into the development of spectral based multivariate models so as to maximize the stability and robustness of these models for the subsequent measurement of property and composition data for unknowns. CPSA allows signals in the calibration spectra which are due to the spectral measurement process rather than the sample components to be modeled such that the resultant predictive models can be constrained to be insensitive to these measurement process signals. The measurement process variables for which constraints are developed also server as quality control variables for monitoring the status of the measurement process during the subsequent application of the model for measurement of unknowns.

A corresponding modification can also be made to the PLS and MLR techniques, which, as explained above, are alternatives to PCA (or PCR). The respective modifications will be referred to herein as Constrained Partial Least Squares (CPLS), and Constrained Multiple Linear Regression (CMLS). Generically, CPSA, CPLS and CMLR will be referred to as Constrained Spectral Analysis (CSA).

The mid- and near-infrared spectra of a molecule consist of numerous absorption bands corresponding to different types of molecular vibrations. In a complex mixture, the absorptions due to a specific molecular type will all vary together in intensity as the concentration of the species changes. The fact that the intensities of these multiple absorptions are correlated in the frequency (wavelength) domain allows them to be distinguished for random noise that is uncorrelated in the frequency domain. Multivariate data analysis methods such as Principal Components Analysis or Partial Least Squares are used to identify and isolate the frequency correlated signals among a series of calibration spectra. These spectral variables can be regressed against properties and component concentration data for the calibration samples to develop a predictive model. During the analysis of unknowns, the spectra are decomposed in terms of these same spectral variables, and regression relationships are used to predict the property/composition of the new samples.

In all real spectral measurements, the variation in sample component concentrations is generally not the only source of frequency correlated signal. Signals arising from the measurement process (e.g. the instrument, the cell, etc.) are superimposed on the absorptions due to the sample components. To the mathematics, these measurement process signals are indistinguishable from the sample component absorptions, and are thus extracted as spectral variables. If these measurement process variables are correlated to the property/concentration (or errors in these values) during the regression, predictions based on the resultant model will be dependent on variations in the measurement process. CPSA allows the spectroscopist to model potential sources of measurement process signals and remove them as spectral variables prior to the regression. The resultant predictive models are constrained to be insensitive to the measurement process signals, and are thus more stable and robust to variations in the spectral data collection.

Examples included in this report demonstrate the use of CPSA for developing predictive models, and compare the CPSA results to those obtained via other multivariate methods.

## Introduction

Principal Components Regression and Partial Least Squares multivariate data analyses are used to correlate the molecular information inherent to spectral data to property and compositional variables. Both PCR and PLS are most often applied to under-determined calibrations, i.e. to calibrations where the number of data points per spectrum exceeds the number of calibration samples. Therefore, both methods require a variable reduction which reduces the dimensionality of the spectral data. While the algorithms used to extract the Principal Components and PLS latent variables differ in computational methodology, both are based on an assumption that there are only two sources of variance in the spectral data. Real components are assumed to give rise to multiple signals whose intensities are linearly correlated in the frequency (or wavelength) domain. Random spectral noise is assumed to be uncorrelated in the frequency domain. The algorithms are designed to isolate the signals that are correlated from the random noise so as to produce spectral variables that can be regressed against concentration and/or property data to produce a predictive model. If the signals due to sample components were the only source of frequency correlated signal in the spectral data, both computational methods would yield stable, robust predictive models which could be used for the subsequent analysis of unknown materials. Unfortunately, in all real spectral measurements, there are additional sources of signals whose intensities are linearly correlated in the frequency domain, which contribute to the total spectral variance, and which are associated with the measurement process rather than the samples. For mid-infrared spectroscopy, examples of the measurement process signals would include reflectance/scattering loses from cell windows, and spectral interferences due to trace water and carbon dioxide in the spectrometer purge gas. If these measurement related signals were constant, they would have no effect on the predictive models. However, since these measurement process signals are themselves subject to variation among real spectra, they are, to the mathematics, indistinguishable from the sample component signals, they can be extracted along with the sample component variations during the variable reduction, and they may be correlated to the properties or component concentrations (or to errors in these dependent values) in the generation of the predictive model. The resultant model will then not be stable and robust with respect to changes in the measurement process related signals.

Spectral preprocessing is used to minimize the effect of measurement related variance on multivariate models. Baseline correction algorithms are an example of commonly employed preprocessing. Data points at which minimal sample component intensity is expected are selected and fit to a "baseline function" (a constant offset, or frequency dependent polynomial). The "baseline" is subtracted from the spectrum prior to the multivariate calibration or analysis.

Spectral subtraction algorithms are also be employed in preprocessing, for the removal of some types of spectral interferences (e.g. water vapor). The objective of the preprocessing is to remove the measurement process related signals from the spectral data prior to the variable reduction. There are several disadvantages to the preprocessing methods that are typically employed for spectral analysis.

(1) The calculations used in preprocessing the spectral data are generally based on a selected subset of the spectral data (e.g. a single point offset correction, or a two point linear baseline correction), and are sensitive to the noise characteristics of the points used for the correction. Depending on the relative magnitudes of the noise and the signal being removed, the preprocessing may be ineffective in removing the measurement process signals, or may only substitute one source of variation (correlated to the spectral noise) for another (the signal being subtracted).

(2) For some types of signals (e.g. spectral interferences which are not well resolved from sample signals, or higher order background terms), effective, reproducible preprocessing algorithms are difficult to develop.

(3) The spectral variables which are removed by the preprocessing step are not orthogonal to the variables (Principal Components) defined by the multivariate analysis. The lack of orthogonality makes it difficult to statistically define the effects of the preprocessing on the resultant model.

(4) Since by definition, the preprocessing is applied before the multivariate analysis, the preprocessing algorithms are not generally included as an integral part of most multivariate programs. The user is thus left with the job of developing and implementing his own preprocessing algorithms and integrating them with the multivariate analysis programs. Since different algorithms can be required to handle different types of measurement process signals, and since the variables that are removed by each algorithm are not orthogonal, the ultimate effect of the preprocessing on the stability and robustness of the predictive model become increasingly difficult to determine.

The purpose of this description is to describe an algorithm which is designed to incorporate preprocessing as an integral part of the multivariate analysis. The Constrained Principal Spectra Analysis (CPSA) algorithm allows the user to model (e.g. provide an example spectrum of) potential sources of measurement related signals. The algorithm generates a set of orthonormal correction variables (spectra) which are removed from the spectral data prior to the multivariate variable reduction. The multivariate predictive model generated in this manner is **constrained** so as to be insensitive (orthogonal) to the presence of these measurement process signals in the spectral data. Since the algorithm uses the entire spectral range in generating the constraints, it is relatively insensitive to spectral noise. The algorithm can correct for polynomial backgrounds as well as poorly resolved spectral interferences. Since the "preprocessing" is an integral part of the algorithm, the effects of including corrections on the resultant predictive model can be readily tested. Finally, the correction variables defined by the CPSA algorithm serve a useful quality control variables for monitoring the measurement process during analysis of unknowns.

Although the **Constraint** algorithm described here is specifically applied as a variation of a Principal Components Analysis, the same methodology could be used to develop a constrained version of the Partial Least Squares analysis.

## Mathematical Basis for CPSA

The object of Principal Components Analysis (PCA) is to isolate the true number of independent variables in the spectral data so as to allow for a regression of these variables against the dependent property/composition variables. The spectral data matrix, **X**, contains the spectra of the *n* samples to be used in the calibration as columns of length *f,* where *f* is the number of data points (frequencies or wavelengths) per spectrum. The object of PCA is to decompose the *f by n* **X** matrix into the product of several matrices. This decomposition can be accomplished via a Singular Value Decomposition:

$$X = U\Sigma V^t \qquad (1)$$

where **U** (the left eigenvector matrix) is of dimension *f* by *n,* $\Sigma$ (the diagonal matrix containing the singular values a) is of dimension *n* by *n,* and $V^t$ is the transpose of **V** (the right eigenvector matrix) which is of dimension *n* by *n.* Since some versions of PCA perform the Singular Value Decomposition on the transpose of the data matrix, $X^t$, and decompose it as $V\Sigma U^t$, the use of the terms left and right eigenvectors is somewhat arbitrary. To avoid confusion, **U** will be referred to as the **eigenspectrum** matrix since the individual column-vectors of **U** (the **eigenspectra**) are of the same length, *f,* as the original calibration spectra. The term **eigenvectors** will only be used to refer to the **V** matrix. The matrices in the singular value decomposition have the following properties:

$$U^t U = I_n \qquad (2)$$

$$VV^t = V^t V = I_n \qquad (3)$$

$$X^t X = V \Lambda V^t \text{ and } XX^t = U \Lambda U^t \qquad (4)$$

where $I_n$ is the $n$ by $n$ identify matrix, and $\Lambda$ is the matrix containing the eigenvalues, $\lambda$ (the squares of the singular values), on the diagonal and zeros off the diagonal. Note that the product $UU^t$ does not yield an identity matrix for $n$ less than $f$. Equations 2 and 3 imply that both the **eigenspectra** and **eigenvectors** are orthonormal. In some version of PCA, the **U** and $\Sigma$ are matrices are combined into a single matrix. In this case, the eigenspectra are orthogonal but are normalized to the singular values.

The object of the variable reduction is to provide a set of independent variables (the Principal Components) against which the dependent variables (the properties or compositions) can be regressed. The basic regression equation for **direct calibration** is

$$Y = X^t P \qquad (5)$$

where **Y** is the $n$ by $c$ matrix containing the property/composition data for the $n$ samples and $c$ properties/components, and **P** is the $f$ by $c$ matrix of regression coefficients which relate the property/composition data to the spectral data. We will refer to the $c$ columns of **P** as **prediction vectors**, since during the analysis of a spectrum **x** (dimension $f$ by 1), the prediction of the properties/components ($y$ of dimension 1 by $c$) for the sample is obtained by

$$y = x^t P \qquad (6)$$

Note that for a single property/component, the prediction is obtained as the dot product of the spectrum of the unknown and the **prediction vector**. The solution to equation 5 is

$$[X^t]^{-1} Y = [X^t]^{-1} X^t P = P \qquad (7)$$

where $[X^t]^{-1}$ is the inverse of the $X^t$ matrix. The matrix $X^t$ is of course non-square and rank deficient ($f>n$), and cannot be directly inverted. Using the singular value decompositions, however, the inverse can be approximated as

$$[X^t]^{-1} = U \Sigma^{-1} V^t \qquad (8)$$

where $\Sigma^{-1}$ is the inverse of the square singular value matrix and contains $1/\sigma$ on the diagonal. Using equations 7 and 8, the **prediction vector** matrix becomes

$$P = U \Sigma^{-1} V^t Y \qquad (9)$$

As was noted previously, the objective of the PCA is to separate systematic (frequency correlated) signal from random noise. The **eigenspectra** corresponding to the larger singular values represent the systematic signal, while those corresponding to the smaller singular values represent the noise. In general, in developing a stable model, these noise components will be eliminated from the analysis before the **prediction vectors** are calculated. If the first $k<n$ **eigenspectra** are retained, the matrices in equation 1 become U' (dimension $f$ by $k$), $\Sigma'$ (dimension $k$ by $k$) and **V'** (dimension $n$ by $k$).

$$X = U' \Sigma' V'^t + E \qquad (10)$$

where $\mathbf{E}$ is an $f$ by $n$ error matrix. Ideally, if all the variations in the data due to sample components are accounted for in the first $k$ eigenspectra, $\mathbf{E}$ contains only random noise. It should be noted that the product $\mathbf{V'V'^t}$ no longer yields an identity matrix. To simplify notation the ' will be dropped, and $\mathbf{U}, \Sigma$ and $\mathbf{V}$ will henceforth refer to the rank reduced matrices. The choice of $k$, the number of eigenspectra to be used in the calibration, is based on statistical tests and some prior knowledge of the spectral noise level.

Although the prediction of a property/component requires only a single **prediction vector**, the calculation of uncertainties on the prediction require the full rank reduced $\mathbf{V}$ matrix. In practice, a two step, **indirect calibration** method is employed in which the singular value decomposition of the $\mathbf{X}$ matrix is calculated (equation 1), and then the properties/compositions are separately regressed against the eigenvectors

$$Y = VB + E \tag{11}$$

$$B = V^tY \tag{12}$$

During the analysis, the eigenvector for the unknown spectrum is obtained

$$v = x^tU\Sigma^{-1} \tag{13}$$

and the predictions are made as

$$y = vB \tag{14}$$

The indirect method is mathematically equivalent to the direct method of equation 10, but readily provides the values needed for estimating uncertainties on the prediction.

Equation 6 shows how the **prediction vector**, $\mathbf{P}$, is used in the analysis of an unknown spectrum. We assume that the unknown spectrum can be separated as the sum of two terms, the spectrum due to the components in the unknown, $\mathbf{x_c}$, and the measurement process related signals for which we want to develop constraints, $\mathbf{x_s}$, The prediction then becomes

$$y = x^tP = x_c^tP + x_s^tP \tag{15}$$

If the prediction is to be insensitive to the measurement process signals, the second term in equation 15 must be zero. This implies that the **prediction vector** must be orthogonal to the measurement process signal spectra. From equation 10, the **prediction vector** is a linear combination of the **eigenspectra**, which in turn are themselves linear combination of the original calibration spectra ($\mathbf{U = XV\Sigma^{-1}}$). If the original calibration spectra are all orthogonalized to a specific measurement process signal, the resulting prediction vector will also be orthogonal, and the prediction will be insensitive to the measurement process signal. This orthogonalization procedure serves as the basis for the Constrained Principal Spectra Analysis algorithm.

In the **C**onstrained **P**rincipal **S**pectra **A**nalysis (CPSA) program, two types of measurement process signals are considered. The program internally generates a set of orthonormal, frequency dependent polynomials, $\mathbf{U_p}$. $\mathbf{U_p}$ is a matrix of dimension $f$ by $p$ where $p$ is the maximum order (degree minus one) of the polynomials, and it contains columns which are orthonormal Legendre polynomials defined over the spectral range used in the analysis. The polynomials are intended to provide constraints for spectral baseline effects. In addition, the user may supply spectra representative of other measurement process signals (e.g. water vapor spectra). These **correction spectra** (a matrix $\mathbf{X_s}$ of dimension $f$ by s where s is the number of correction spectra) which may include multiple examples of a specific type of measurement process signal, are first orthogonalized relative to the polynomials via a Gram-Schmidt orthogonalization procedure

$$X_s' = X_s - U_p(U_p^tX_s) \tag{16}$$

A Singular Value Decomposition of the resultant correction spectra is then performed,

$$X_s{}' = U_s \Sigma_s V_s{}^t \tag{17}$$

to generate a set of orthonormal correction eigenspectra, $\mathbf{U_s}$. The user selects the first $s'$ terms corresponding to the number of measurement related signals being modeled, and generates the full set of correction terms, $\mathbf{U_m}$, which includes both the polynomials and selected correction eigenspectra. These correction terms are then removed from the calibration data, again using a Gram-Schmidt orthogonalization procedure

$$X_c = X - U_m(U_m{}^t X) \tag{17}$$

The Principal Components Analysis of the corrected spectra, $\mathbf{X_c}$, then proceeds via the Singular Value Decomposition

$$X_c = U_c \Sigma_c V_c{}^t \tag{18}$$

and the predictive model is developed using the regression

$$Y = V_c B \tag{19}$$

The resultant **prediction vector**

$$P_c = U_c \Sigma_c{}^{-1} V_c{}^t Y \tag{20}$$

is orthogonal to the polynomial and correction eigenspectra, $U_m$. The resulting predictive model is thus insensitive to the modeled measurement process signals. In the analysis of an unknown, the contributions of the measurement process signals to the spectrum can be calculated as

$$v_m = \Sigma_m{}^{-1} U_m{}^t x \tag{21}$$

and these values can be compared against the values for the calibration, $\mathbf{V_m}$, to provide diagnostic as to whether the measurement process has changed relative to the calibration.

The results of the procedure described above are mathematically equivalent to including the polynomial and correction terms as spectra in the data matrix, and using a constrained least square regression to calculate the B matrix in equation 12. The constrained least square procedure is more sensitive to the scaling of the correction spectra since they must account for sufficient variance in the data matrix to be sorted into the $k$ **eigenspectra** that are retained in the regression step. By orthogonalizing the calibration spectra to the correction spectra before calculating the singular value decomposition, we eliminate the scaling sensitivity.

The Constrained Principal Spectra Analysis method allows measurement process signals which are present in the spectra of the calibration samples, or might be present in the spectra of samples which are latter analyzed, to be modeled and removed from the data (via a Gram-Schmidt orthogonalization procedure) prior to the extraction of the spectral variables which is performed via a Singular Value Decomposition (16). The spectral variables thus obtained are first regressed against the pathlengths for the calibration spectra to develop a model for independent estimation of pathlength. The spectral variables are resealed to a common pathlength based on the results of the regression and then further regressed against the composition/property data to build the empirical models for the estimation of these parameters. During the analysis of new samples, the spectra are collected and decomposed into the constrained spectral variables, the pathlength is calculated and the data is scaled to the appropriate pathlength, and then the regression models are applied to calculate the composition/property data for the new materials. The orthogonalization procedure ensures that the resultant measurements are constrained so as to be insensitive (orthogonal) to the modeled measurement process signals. The internal pathlength calculation and renormalization automatically corrects for pathlength or flow variations, thus minimizing errors due to data scaling.

The development of the empirical model consists of the following steps:

(1.1) The properties and/or component concentrations for which empirical models are to be developed are independently determined for a set of representative samples, e.g the calibration set. The independent measurements are made by standard analytical tests including, but not limited to: elemental compositional analysis (combustion analysis, X-ray fluorescence, broad line NMR); component analysis (gas chromatography, mass spectroscopy); other spectral measurements (IR, UV/visible, NMR, color); physical property measurements (API or specific gravity, refractive index, viscosity or viscosity index); and performance property measurements (octane number, cetane number, combustibility). For chemicals applications where the number of sample components is limited, the compositional data may reflect weights or volumes used in preparing calibration blends.

(1.2) Absorption spectra of the calibration samples are collected over a region or regions of the infrared, the data being digitized at discrete frequencies (or wavelengths) whose separation is less than the width of the absorption features exhibited by the samples.

(2.0) The **C**onstrained **P**rincipal **S**pectra **A**nalysis (CPSA) algorithm is applied to generate the empirical model. The algorithm consists of the following 12 steps:

(2.1) The infrared spectral data for the calibration spectra is loaded into the columns of a matrix **X**, which is of dimension $f$ by $n$ where $f$ is the number of frequencies or wavelengths in the spectra, and $n$ is the number of calibration samples.

(2.2) Frequency dependent polynomials, $U_p$, (a matrix whose columns are orthonormal Legendre polynomials having dimension $f$ by $p$ where $p$ is the maximum order of the polynomials) are generated to model possible variations in the spectral baseline over the spectral range used in the analysis.

(2.3) Spectra representative of a other types of measurement process signals (e.g. water vapor spectra, carbon dioxide, etc.) are loaded into a matrix $X_s$ of dimension $f$ by $s$ where $s$ is the number of correction spectra used.

(2.4) The correction spectra are orthogonalized relative to the polynomials via a Gram-Schmidt orthogonalization procedure

$$X_s' = X_s - U_p(U_p{}^t X_s) \tag{2.4}$$

(2.5) A Singular Value Decomposition of the correction spectra is then performed,

$$X_s' = U_s \Sigma_s V_s{}^t \tag{2.5}$$

to generate a set of orthonormal correction eigenspectra, $U_s$. $\Sigma_s$ are the corresponding singular values, and $V_s$ are the corresponding right eigenvectors, $^t$ indicating the matrix transpose.

(2.6) The full set of correction terms, $U_m = U_p + U_s$, which includes both the polynomials and correction eigenspectra are then removed from the calibration data, again using a Gram-Schmidt orthogonalization procedure

$$X_c = X - U_m(U_m{}^t X) \tag{2.6}$$

(2.7) The Singular Value Decomposition of the corrected spectra, $X_c$, is then performed

$$X_c = U_c \Sigma_c V_c{}^t \tag{2.7}$$

(2.8) The eigenspectra from step (2.7) are examined and the a subset of the first $k$ eigenspectra which correspond to the larger singular values in $\Sigma_c$ are retained. The $k+1$ through $n$ eigenspectra which correspond to spectral noise are discarded.

$$X_c = U_k \Sigma_k V_k{}^t + E_k \tag{2.8}$$

(2.9) The $k$ right eigenvectors from the singular value decomposition, $V_k$, are regressed against the pathlength values for the calibration spectra, $Y_p$ (an $n$ by 1 row vector),

$$Y_p = V_k B_p + E_p \tag{2.9a}$$

where $\mathbf{E_p}$ is the regression error. The regression coefficients, $\mathbf{B_p}$, are calculated as

$$B_p = (V_k{}^tV_k)^{-1}V_k{}^tY_p = V_k{}^tY_p \qquad (2.9b)$$

(2.10) An estimation of the pathlengths for the calibration spectra is calculated as

$$\hat{Y}_p = V_kB_p \qquad (2.10)$$

A $n$ by $n$ diagonal matrix $\mathbf{N}$ is then formed, the $i^{th}$ diagonal element of $\mathbf{N}$ being the ratio of the average pathlength for the calibration spectra, $\bar{\mathbf{y}}_{\mathbf{p}}$, divided by the estimated pathlength values for the $i^{th}$ calibration sample (the $i^{th}$ element of $\hat{\mathbf{Y}}_{\mathbf{p}}$).
(2.11) The right eigenvector matrix is then renormalized as

$$V_k{}' = NV_k \qquad (2.11)$$

(2.12) The renormalized matrix is regressed against the properties and or concentrations, $\mathbf{Y}$ ($\mathbf{Y}$, a $n$ by $c$ matrix containing the values for the $n$ calibration samples and the $c$ property/concentrations) to obtain the regression coefficients for the models,

$$Y = V_k{}'B + E \qquad (2.12a)$$

$$B = (V_k{}'^tV_k{}')^{-1}V_k{}'Y \qquad (2.12b)$$

(3.0) The analysis of a new sample with unknown properties/components proceeds by the following steps:
(3.1) The absorption spectrum of the unknown is obtained under the same conditions used in the collection of the calibration spectra.
(3.2) The absorption spectrum, $\mathbf{x_u}$, is decomposed into the constrained variables,

$$x_u = U_k\Sigma_kv_u{}^t \qquad (3.2a)$$

$$v_u = \Sigma^{-1}U_k{}^tx_u \qquad (3.2b)$$

(3.3) The pathlength for the unknown spectrum is estimated as

$$\hat{y}_p = v_uB_p \qquad (3.3)$$

(3.4) The eigenvector for the unknown is resealed as

$$v_u{}' = v_u(\bar{y}_p/\hat{y}_p) \qquad (3.4)$$

where $\bar{\mathbf{y}}_{\mathbf{p}}$ is the average pathlength for the calibration spectra in (2.10).
(3.5) The properties/concentrations are estimated as

$$\hat{y}_u = v_u{}'B \qquad (3.5)$$

(4.1) The spectral region used in the calibration and analysis may be limited to subregions so as to avoid intense absorptions which may be outside the linear response range of the spectrometer, or to avoid regions of low signal content and high noise.

(5.1) The samples used in the calibration may be restricted by excluding any samples which are identified as multivariate outliers by statistical testing.

(6.1) The regression in steps (2.9) and (2.12) may be accomplished via a step-wise regression (see, for example, W.J. Kennedy and J.E. Gentle, Statistical Computing, Marcel Dekker, New York, 1980) or PRESS based variable selection (see, for example, D.M. Allen, **Technical Report Number 23**, University of Kentucky Department of Statistics, August 1971), so as to limit the number of variables retained in the empirical model to a subset of the first *k* variables, thereby eliminating variables which do not show statistically significant correlation to the parameters being estimated.

(7.1) The Mahalanobis statistic for the unknown, $D_u^2$, given by

$$D_u^2 = v_u{'}(V_k{'}^t V_k{'})^{-1} v_u{'}^t \tag{7.1}$$

can be used to determine if the estimation is based on an interpolation or extrapolation of the model by comparing the value for the unknown to the average of similar values calculated for the calibration samples.

(7.2) The uncertainty on the estimated value can also be estimated based on the standard error from the regression in (2.12) and the Mahalanobis statistic calculated for the unknown.

(8.1) In the analysis of an unknown with spectrum $x_u$, the contributions of the measurement process signals to the spectrum can be calculated as

$$v_m = \Sigma_m^{-1} U_m^t x_u \tag{8.1}$$

These values can be compared against the values for the calibration, $V_m$, to provide diagnostics as to whether the measurement process has changed relative to the calibration.

## EXAMPLES

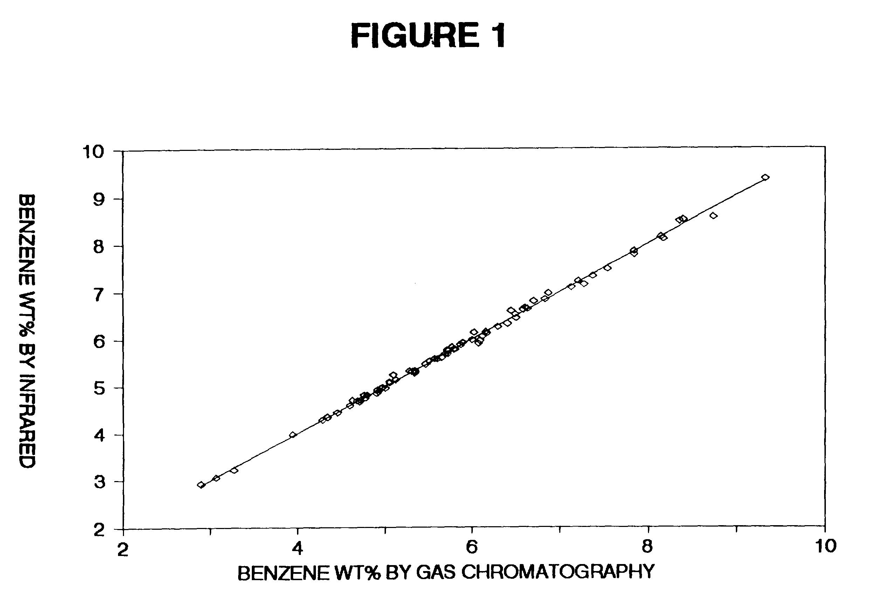

Examples of the use of the CPSA method described above for the generation of empirical models are provided for cases illustrating the use of different portions of the infrared region, and the estimation of compositions as well as physical and performance properties. These examples are identified as Examples 1 to 5 below and the results are illustrated in Figures 1 to 5 respectively. These figures demonstrate the validity of the present method of estimating property and composition data.

**Example 1 - Estimation of a Component Concentration using Mid-Infrared:**

| | |
|---|---|
| Parameter estimated | Weight percent benzene |
| Sample types | Powerformates |
| Calibration samples measured by | Gas Chromatography |
| Spectrometer used | Mattson Polaris/Icon |
| Average Pathlength Calibration set | 500 microns |
| Spectral range used | 5000 - 1645 cm$^{-1}$ |
| Excluded subregions | 3150-2240 cm$^{-1}$ |
| Constraints used | 3 polynomial terms (quadratic) Water vapor spectrum |
| Regression method used | PRESS |
| Number of calibration spectra | 77 |
| # eigenspectra used in regressions *(k)* | 5 |
| # retained in pathlength regression | 4 |
| Standard Error Pathlength Regression | 1.272 microns |
| # retained in composition regression | 5 |
| Standard Error composition regression | 0.063 weight percent |

A Constrained Principal Components model was constructed for the estimation of benzene content of powerformates. The spectra of 77 reference powerformates were collected over the 5000 to 1645 $cm^{-1}$ region using a Mattson Polaris/Icon FT-IR spectrometer operating at 2 $cm^{-1}$ resolution and employing 100 scans for signal averaging. 500 micron calcium floride cells were employed. To avoid ranges where the sample absorption was beyond the linear response range of the spectrometer, and to avoid the need for adding a carbon dioxide correction term to the model, the data in the 3150-2240 $cm^{-1}$ region were excluded during the model generation. Benzene contents for the reference samples used in the calibration were obtained via gas chromatographic analysis. A CPSA model was developed using 3 polynomial correction terms to account for possible background variations, and a water vapor correction spectrum to account for possible purge variations. A PRESS based step-wise regression (see, for example, the above mentioned D.M. Allen reference) was employed for developing both a pathlength estimation model, and the benzene content. Of the 5 Constrained Principal Component variable input into the PRESS regression, 4 were retained for the pathlength estimation, and all 5 were retained for the benzene estimation. The standard error for the estimation of the cell pathlength was 1.272 microns, and the standard error for the estimation of the benzene content was 0.063 weight percent. A plot of the infrared estimated benzene content versus that measured by GC for the 77 reference samples is shown in Figure 1.

**Example 2 - Physical Property Estimation by Mid-Infrared:**

| | |
|---|---|
| Parameter estimated | API Gravity |
| Sample types | Petroleum mid-distillates |
| Calibration samples measured by | ASTM D1298 |
| Spectrometer used | Mattson Polaris/Icon |
| Average Pathlength Calibration set | 29.57 microns |
| Spectral range used | 3650 - 500 $cm^{-1}$ |
| Excluded subregions | 2989-2800 $cm^{-1}$, |
| | 2400-2300 $cm^{-1}$, |
| | 1474-1407 $cm^{-1}$ |
| Constraints used | 3 polynomial terms |
| | (quadratic) |
| | Water vapor spectrum |
| Regression method used | PRESS |
| Number of calibration spectra | 91 |
| # eigenspectra used in regressions ($k$) | 24 |
| # retained for pathlength regression | 21 |
| Standard Error Pathlength Regression | 0.159 microns |
| # retained in composition regression | 21 |
| Standard Error composition regression | 0.660 degrees API |

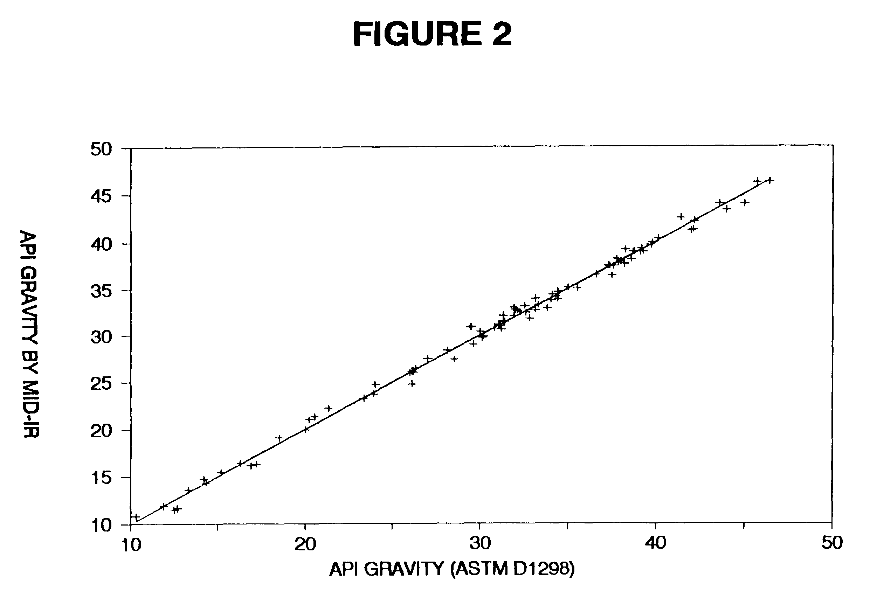

A Constrained Principal Components model was constructed for the estimation of API Gravity for petroleum mid-distillates. The spectra of 91 reference mid-distillates were collected over the 3650 to 500 $cm^{-1}$ region using a Mattson Polaris/Icon FT-IR spectrometer operating at 2 $cm^{-1}$ resolution and employing 100 scans for signal averaging. 30 micron potassium bromide cells were employed. To avoid ranges where the sample absorption was beyond the linear response range of the spectrometer, and to avoid the need for adding a carbon dioxide correction term to the model, the data in the 2989-2800 $cm^{-1}$, 2400-2300 $cm^{-1}$ and 1474-1407 $cm^{-1}$ regions was excluded during the model generation. API Gravities for the reference samples used in the calibration were obtained via ASTM D1298. A CPSA model was developed using 3 polynomial correction terms to account for possible background variations, and a water vapor correction spectrum to account for possible purge variations. A PRESS based step-wise regression (see, for example, the above mentioned D.M. Allen reference) was employed for developing both a pathlength estimation model, and the API Gravity model. Of the 24 Constrained Principal Component variable input into the PRESS regression, 19 were retained for the pathlength estimation, and 21 were retained for the API Gravity estimation. The standard error for the estimation of the cell pathlength was 0.159 microns, and the standard error for the estimation of the API Gravity was 0.660 degrees API. A plot of the infrared estimated API Gravity versus that measured by ASTM D1298 for the 91 reference samples is shown in Figure 2.

**Example 3 - Estimation of a Performance Property using Near-Infrared:**

| Parameter estimated | Cetane number |
|---|---|
| Sample types | Petroleum mid-distillates |
| Calibration samples measured by | ASTM D-613 |
| Spectrometer used | Mattson Sirius 100 |
| Average Pathlength Calibration set | 519.3 microns |
| Spectral range used | 10000 - 3800 $cm^{-1}$ |
| Excluded subregions | none |
| Constraints used | 3 polynomial terms (quadratic) |
| Regression method used | PRESS |
| Number of calibration spectra | 93 |
| # eigenspectra used in regressions (k) | 13 |
| # retained for pathlength regression | 11 |
| Standard Error Pathlength Regression | 1.535 microns |
| # retained in composition regression | 10 |
| Standard Error composition regression | 1.258 cetane number |

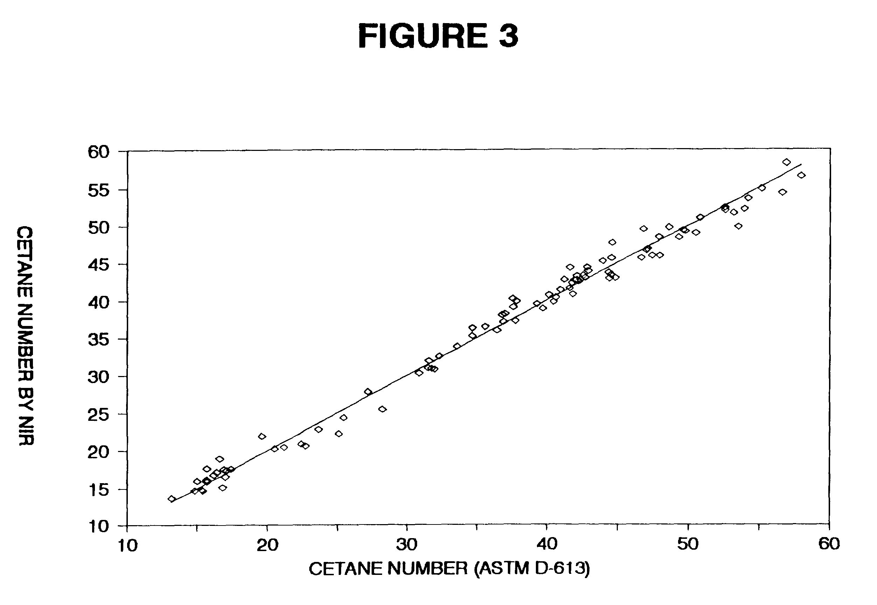

A Constrained Principal Components model was constructed for the estimation of Cetane Number for petroleum mid-distillates. The spectra of 91 reference mid-distillates were collected over the 10000 to 3800 $cm^{-1}$ region using a Mattson Sirius 100 FT-IR spectrometer operating at 2 $cm^{-1}$ resolution and employing 100 scans for signal averaging. 500 micron calcium floride cells were employed. Cetane numbers for the reference samples used in the calibration were obtained via ASTM D-613. A CPSA model was developed using 3 polynomial correction terms to account for possible background variations. A PRESS based step-wise regression (see, for example, the above mentioned D.M. Allen reference) was employed for developing both a pathlength estimation model, and the cetane number model. Of the 13 Constrained Principal Component variable input into the PRESS regression, 11 were retained for the pathlength estimation, and 10 were retained for the cetane number estimation. The standard error for the estimation of the cell pathlength was 1.535 microns, and the standard error for the estimation of the cetane number was 1.258 cetane numbers. A plot of the infrared estimated cetane number versus that measured by ASTM D-613 for the 91 reference samples is shown in Figure 3.

**Example 4 - Elemental Composition Esimation by Mid-Infrared:**

| Parameter estimated | Weight percent hydrogen |
|---|---|
| Sample types | Petroleum mid-distillates |
| Calibration samples measured by | Broad line NMR |
| Spectrometer used | Mattson Polaris/Icon |
| Average Pathlength Calibration set | 29.57 microns |
| Spectral range used | 3650 - 500 $cm^{-1}$ |
| Excluded subregions | 2989-2800 $cm^{-1}$, 2400-2300 $cm^{-1}$, 1474-1407 $cm^{-1}$ |
| Constraints used | 3 polynomial terms (quadratic) Water vapor spectrum |
| Regression method used | PRESS |
| Number of calibration spectra | 91 |
| # eigenspectra used in regressions (k) | 24 |
| # retained for pathlength regression | 19 |
| Standard Error Pathlength Regression | 0.159 microns |
| # retained in composition regression | 21 |

(continued)

| Standard Error composition regression | 0.0551 weight percent |
|---|---|

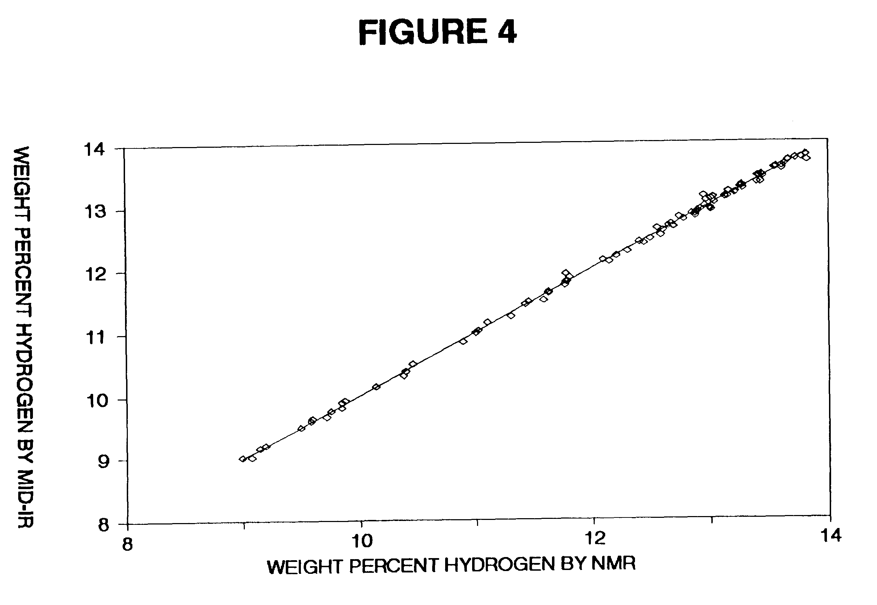

A Constrained Principal Components model was constructed for the estimation of hydrogen content for petroleum mid-distillates. The spectra of 91 reference mid-distillates were collected over the 3650 to 500 cm$^{-1}$ region using a Mattson Polaris/Icon FT-IR spectrometer operating at 2 cm$^{-1}$ resolution and employing 100 scans for signal averaging. 30 micron potassium bromide cells were employed. To avoid ranges where the sample absorption was beyond the linear response range of the spectrometer, and to avoid the need for adding a carbon dioxide correction term to the model, the data in the 2989-2800 cm$^{-1}$, 2400-2300 cm$^{-1}$ and 1474-1407 cm$^{-1}$ regions was excluded during the model generation. Hydrogen contents for the reference samples used in the calibration were obtained via Broad line NMR. A CPSA model was developed using 3 polynomial correction terms to account for possible background variations, and a water vapor correction spectrum to account for possible purge variations. A PRESS based stepwise regression (see, for example, the above mentioned D.M. Allen reference) was employed for developing both a pathlength estimation model, and the hydrogen content model. Of the 24 Constrained Principal Component variable input into the PRESS regression, 19 were retained for the pathlength estimation, and 21 were retained for the hydrogen content estimation. The standard error for the estimation of the cell pathlength was 0.159 microns, and the standard error for the estimation of the hydrogen cotent was 0.0551 weight percent hydrogen. A plot of the infrared estimated hydrogen content versus that measured by broad line NMR for the 91 reference samples is shown in Figure 4.

**Example 5 - Chemical Composition Estimation by Mid-Infrared:**

| | |
|---|---|
| Parameter estimated | Weight percent ZDDP |
| Sample types | Lubricant additive package |
| Calibration samples measured by | Weight % in blends |
| Spectrometer used | Digilab FTS-20C |
| Average Pathlength Calibration set | 62.0 microns |
| Spectral range used | 1800 - 490 cm$^{-1}$ |
| Excluded subregions | 1475 - 1435 cm$^{-1}$ |
| Constraints used | 3 polynomial terms (quadratic) Water vapor spectrum |
| Regression method used | Step-wise |
| Number of calibration spectra | 30 |
| # eigenspectra used in regressions (k) | 7 |
| # retained for pathlength regression | 7 |
| Standard Error Pathlength Regression | 0.17 microns |
| # retained in composition regression | 7 |
| Standard Error composition regression | 0.16 weight percent |

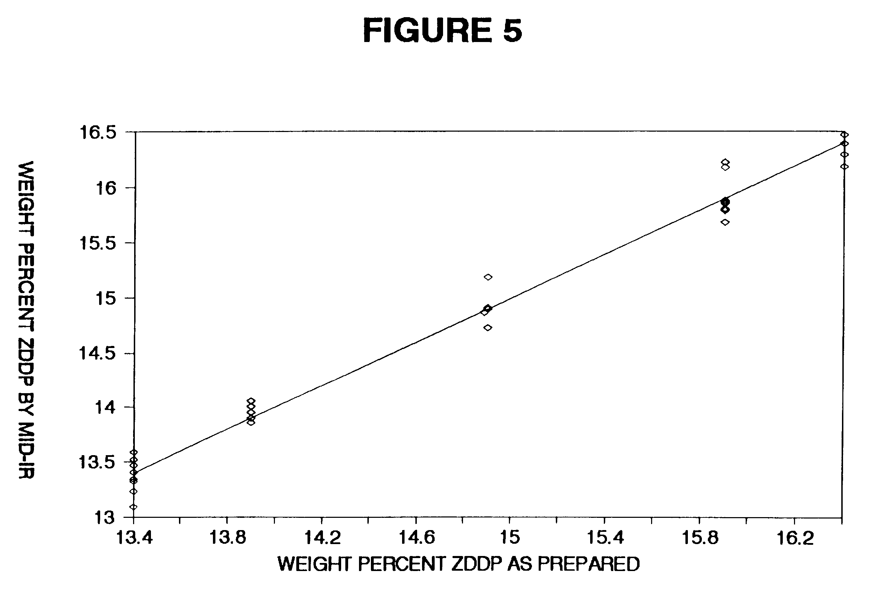

A Constrained Principal Components model was constructed for the estimation of the zinc dialkyl dithiophosphate (ZDDP) content for lubricant additive packages. 30 reference blends of an additive package containing a polyisobutenyl polyamine dipersant, a overbased magnesium sulfonate detergent, a sulfurized nonyl phenol, ZDDP and diluent oil were prepared for the calibration. The additive concentrations in the reference blends were varied at +/- 8 to 12 percent of the target concentrations. Solutions containing 50% additive package in cyclohexane were prepared and spectra were collected over the 3650 to 400 cm$^{-1}$ region using a Digilab FTS-20C FT-IR spectrometer operating at 2 cm$^{-1}$ resolution. 100 scans were employed for signal averaging. 62 micron potassium bromide cells were employed. The CPSA model was developed using spectra data in the 1800-1475 and 1435-490 cm$^{-1}$ regions. A CPCR model was developed using 3 polynomial correction terms to account for possible background variations, and a water vapor correction spectrum to account for possible purge variations. A step-wise regression (see, for example, the above mentioned W.J. Kennedy and J.E. Gentle reference) was employed for developing both a pathlength estimation model, and the ZDDP content. Of the 7 Constrained Principal Component variable input into the PRESS regression, 7 were retained for the pathlength estimation, and 7 were retained for the ZDDP content estimation. The standard error for the estimation of the cell pathlength was 0.17 microns, and the standard error for the estimation of the ZDDP cotent was 0.16 weight percent. A plot of the infrared estimated ZDDP content versus that used in preparing the 30 blends is shown in Figure 5.

Further examples will now be given.

## Example 6 - Binary Blends of Isooctane and Heptane

The first example demonstrates how a Constrained Principal Components Analysis can be used to develop a model that is robust relative to variations in signals arising from the spectral measurement process. Mid-infrared (4000-400 cm$^{-1}$) spectra of 22 binary mixtures of isooctane (2,2,4-trimethyl pentane) and n-heptane were collected on a Mattson Polaris/Icon FT-IR spectrometer at 2 cm$^{-1}$ resolution, using 100 scans for signal averaging and a 25 micron potassium bromide fixed pathlength cell. The single beam sample spectra were ratioed against empty beam background spectra for the calculation of the absorbance spectra. Principal Components Analysis and Constrained Principal Components Analysis were both used to generate models for the estimation of the isooctane and heptane contents of the binary mixtures. To avoid absorbances that might be outside the linear response range of the spectrometer, only the data in the 2000-690 cm$^{-1}$ spectral range were used in the models. The spectra of 11 of the binary mixtures were used in developing the models, and the spectra of the remaining 11 mixtures were used to test the models. The concentrations of the mixtures used for generating and testing the models are given in Table 1.

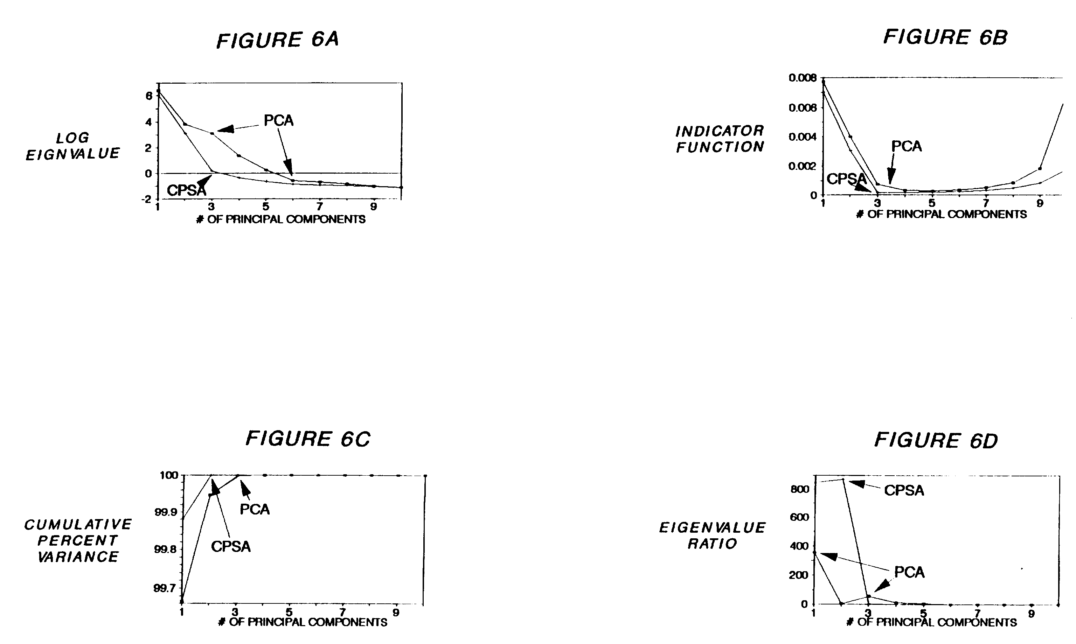

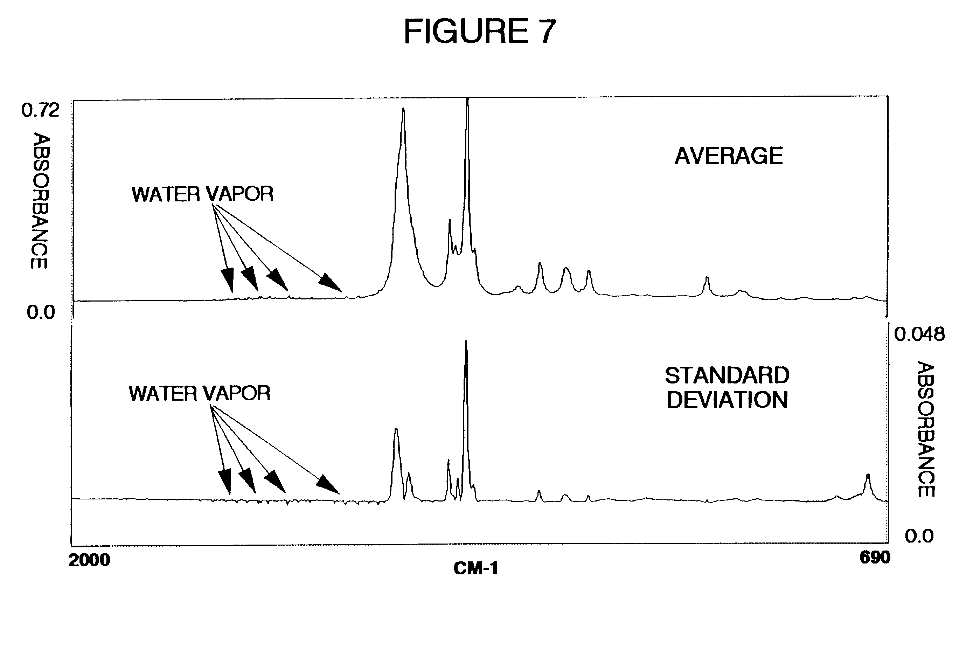

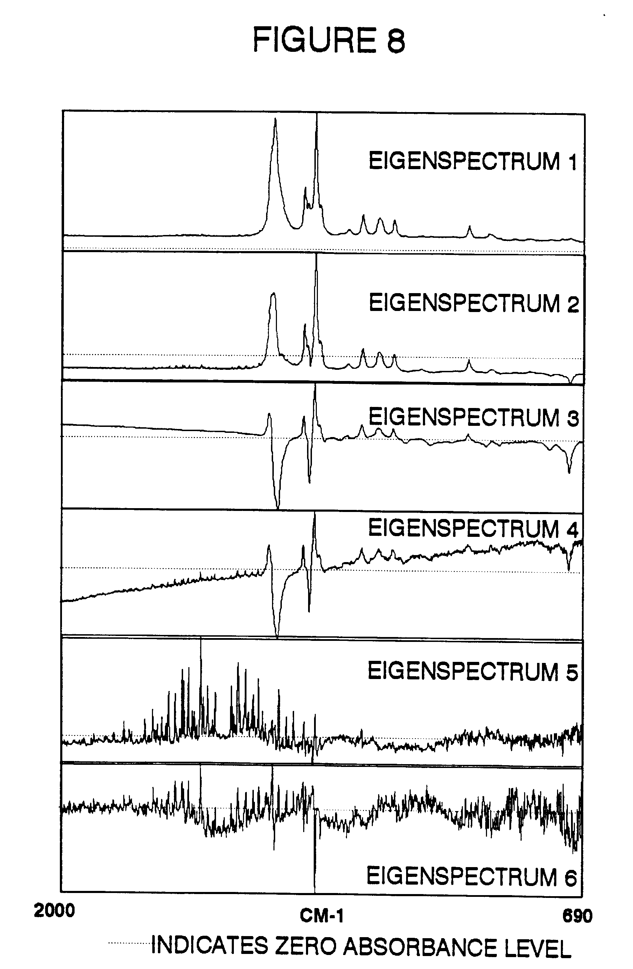

Figure 6 shows the statistics for the Principal Components Analysis (PCA) of the blend spectra. As is typical with real systems, the various statistical tests do not unambiguously indicate the true number of spectral variables. The plot of the logarithm of the eigenvalues versus number of Principal Components decreases relatively smoothly, showing only minor breaks at 3 and 6 Principal Components. The indicator function goes through a minimum at 5 variables, the cumulative variance appear to level off after 3 Principal Components, and the eigenvalue ratios have maxima at 1 and 3 Principal Components. None of the statistics indicates the true number of real components in the binary blends. Examination of the average and standard deviation spectra for the calibration spectra (Figure 7) suggests the sources of these additional spectral variables. There is clearly a frequency dependent variation in the spectral baseline as well as absorptions due to water vapor due to incomplete spectrometer purge. Examination of the eigenspectra produced by the PCA analysis (Figure 8) demonstrates how these additional measurement related variations are extracted as Principal Components. Eigenspectrum 1 shows the absorptions due to the two real components, but is clearly offset relative to the zero absorption. Eigenspectrum 2 shows only slight differentiation between the isooctane and heptane absorptions, and is dominated by an offset. Eigenspectra 3 and 4 differentiate between the two real components, but also show a frequency dependent variation in the spectral background. Eigenspectrum 5 is clearly that of water vapor. Eigenspectrum 6 is largely measurment noise. Note that the measurement process related signals are not cleanly extracted into single Principal Components, but are mixed among all the spectral variables. The offset is clearly present in both Eigenspectra 1 and 2. Water vapor absorptions are observed in Eigenspectra 1,2,4 and 5, and the frequency dependent background is present in Eigenspectra 3 and 4.

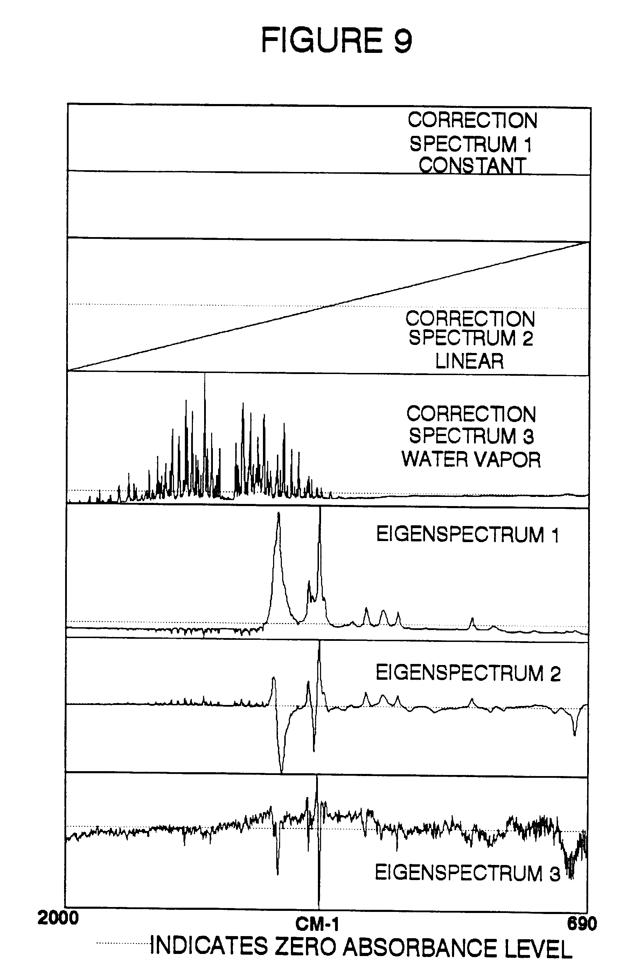

A CPSA model was developed for the same data set, using a second order (constant plus linear) polynomial background correction and water vapor correction spectra as constraints. Figure 9 shows the three correction spectra used, as well as the first three eigenspectra generated by the CPSA analysis. The eigenspectra are now orthogonal to an offset, a linear frequency dependent background and the water vapor correction spectra. The third eigenspectra consists almost exclusively of noise, indicating that the isooctane and heptane spectral variation has been successfully extracted into two spectral variables.

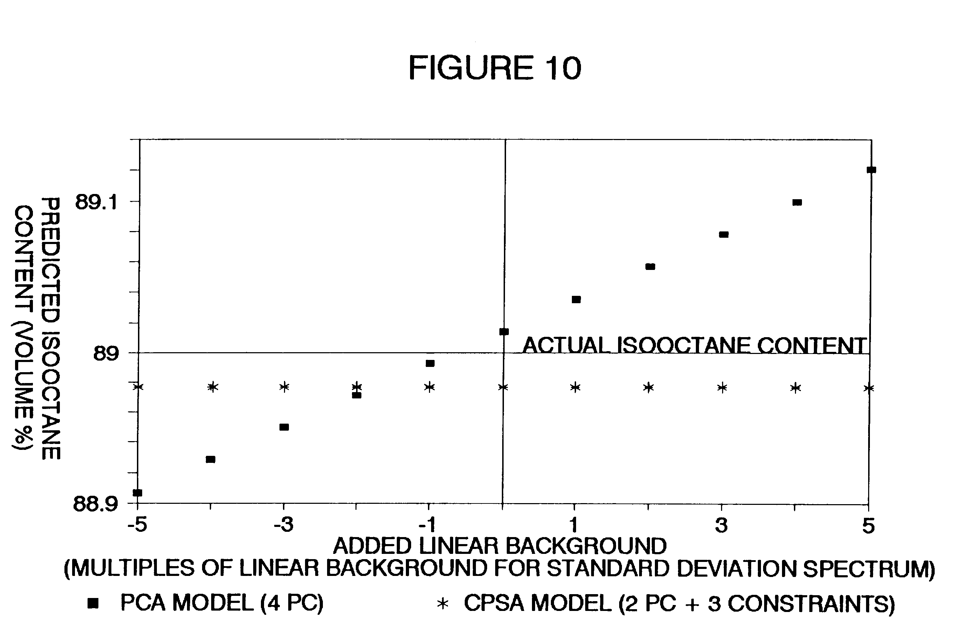

Table 2 shows the Standard Errors for the PCA and CPSA models. As would be expected from the eigenspectra, the PCA model requires 4 variables to account for the variation in the isooctane and heptane concentrations. Inclusion of the fifth Principal Component in the PCA model appears to produce a slight improvement in the Standard Error of Estimate from the calibration, but actually produces a slight degradation of the predictive model. The CPSA model based on two variables has predictive power comparable to the PCA model with 4 variables, but is more robust with respect to the measurement process signals which are present in the spectral data. Figure 10 demonstrates this improved robustness. The variability in the background among the calibration samples was estimated by fitting a linear baseline to the standard deviation spectrum in Figure 7 (calculating the slope and intercept of the line connecting the two endpoints at 2000 and 690 cm$^{-1}$). Multiples of this estimated background were then added to the spectrum of the sample containing 89% isooctane, and the generated spectra were analyzed using the 4 variable PCA model and the 2 variable CPSA model. For the PCA model, the predicted isooctane content clearly depends on the background. Over the range of backgrounds present in the spectral data, variations on the order of 0.05% are observed. If, in the analysis of an unknown, a larger difference in background was present in the spectra, errors on the order of 0.1% could easily be obtained. The CPSA model is independent of the variation in background and produces the same results regardless of the change in background. CPSA thus provides a more robust and stable predictive model.

The major source of error for both the PCR and CPCR analyses is the fact that the sum of the two components does not equal 100%. As is shown in Table 2, if the estimated isooctane and n-heptane concentrations are renormalized to 100%, the SEEs and SEPs are significanlty reduced. Even after renormalization, 4 variables are required to produce a PCR model comparable to the 2 variable CPCR model. It should be noted that, as is shown in Table 2, if the pathlength correction is used in developing the PCR and CPCR models, the renormalization of the estimated isooctane and n-heptane concentrations results automatically from the constraint that the sample components must sum to 100%.

### Example 7 - Analysis of a 5 Component Additive Package Comparison to the K Matrix Method

We have previously demonstrated the use of the **K Matrix** method for the quality control analysis of a oil additive package. In the **K Matrix** analysis, the calibration f by $n$ spectral matrix, **X**, is expressed as the product of two matrices, **K** and **C**.

$$X = KC$$

**C** is a $c$ by $n$ matrix containing the concentrations of the $c$ real components for the $n$ calibration samples. The $f$ by $c$ K matrix is obtained as

$$K = XC^t(CC^t)^{-1}$$

**K** contains the spectra of the real components as they exist in the calibration mixture, i.e. including any intercomponent interactions. The **K** matrix obtained in the calibration can be used in the analysis of a unknown, **x**, to obtain the component concentrations, **c**

$$c = (K^tK)^{-1}K^tx$$

Unlike the Principal Component methods, the use of the **K Matrix** method requires that the concentrations of all the components in the mixtures be known such that the **C** matrix which must be inverted completely specifies the calibration mixtures.

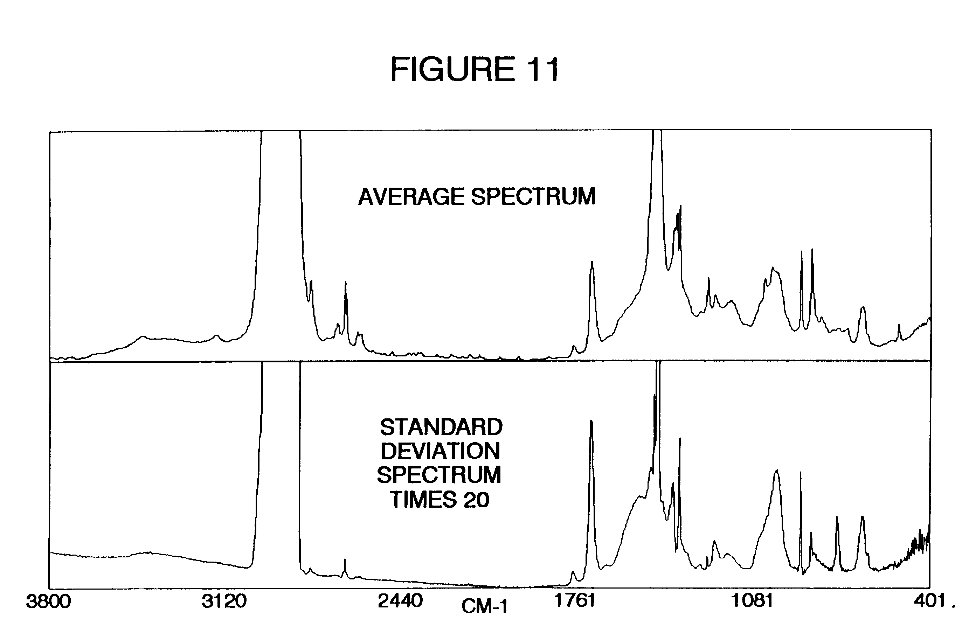

To further demonstrate how CPSA can be used to develop a calibration for a real multicomponent analysis, a more detailed description of the analysis presented in Example 5 will be given to to demonstrate how a predictive model was developed using the same spectral data which had been used for the **K Matrix** analysis. The additive package in question contains five components: a dispersant (49%), a nonyl phenol (NPS, 16%) , a zinc dialkyl-dithiophosphate (ZDDP, 15%) a magnesium overbased sulfonate (12%) and a diluent oil (8%). For the quality control applications, the calibration was developed over a relatively narrow concentration range bracketing the target product composition. 30 blends were prepared by blending the 4 additives at levels corresponding to 88%, 92%, 100%, 108% or 112% of the target concentrations, and adjusting the diluent oil level to achieve the appropriate total weight. The levels for additives in the individual blends were chosen randomly subject to the constraints that the number of times each additive level was represented should be roughly equal, and that all the components vary independently. Spectra of 50% solutions of the blends in cyclohexane were obtained at 2 cm$^{-1}$ resolution using a 0.05 millimeter KBr cell on a Digilab FTS-20C FT-IR spectrometer using 500 scans for signal averaging. 15 of the 30 blends which spanned the additive concentration ranges were chosen for development of the models and the remaining 15 were analyzed to test the models. Figure 11 shows the average and standard deviation spectra for the 30 blends. Spectral data in the range from 1800 to 490 cm$^{-1}$ was used in the analysis. To avoid absorbances that are too strong to accurately measure, the data in the range from 1475 to 1435 cm$^{-1}$ was excluded from the analysis.

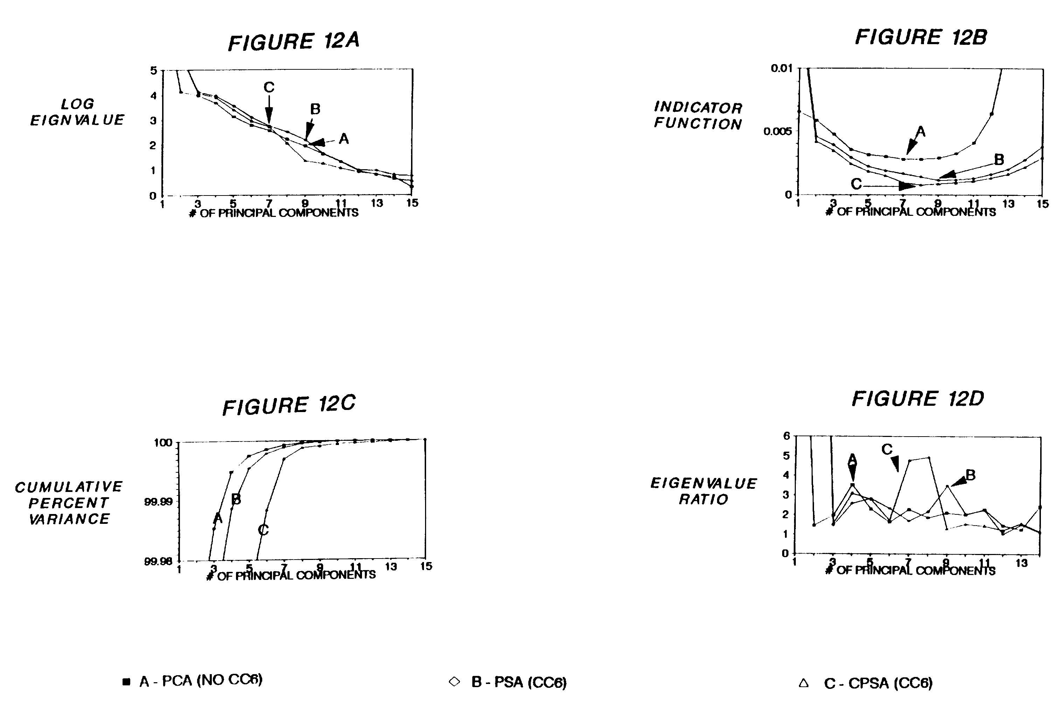

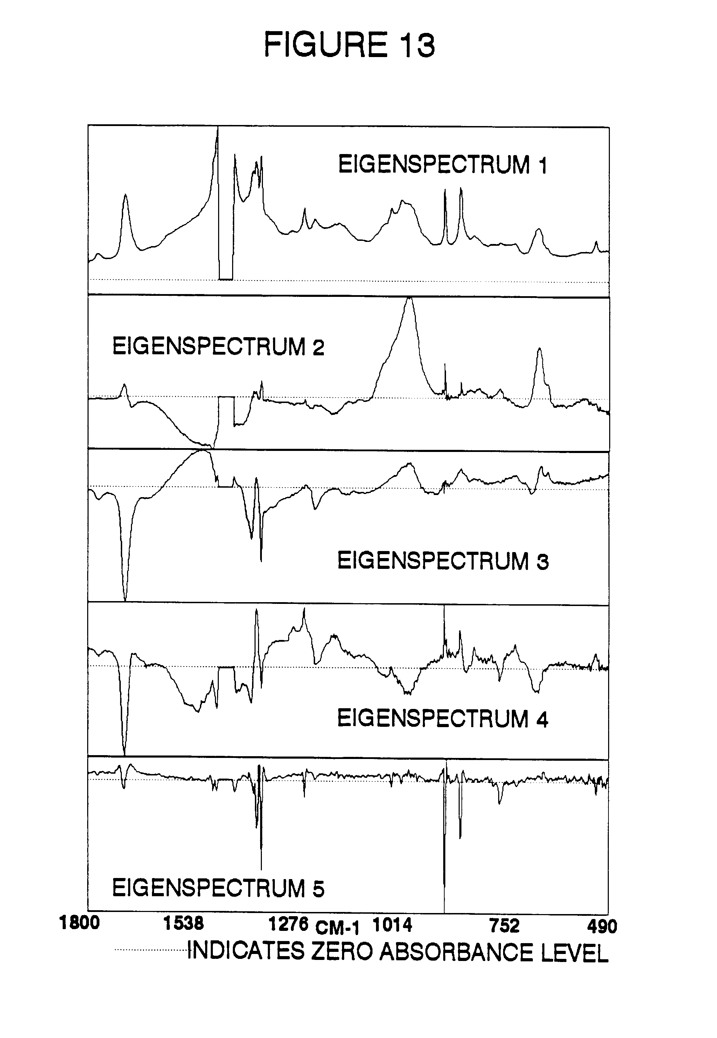

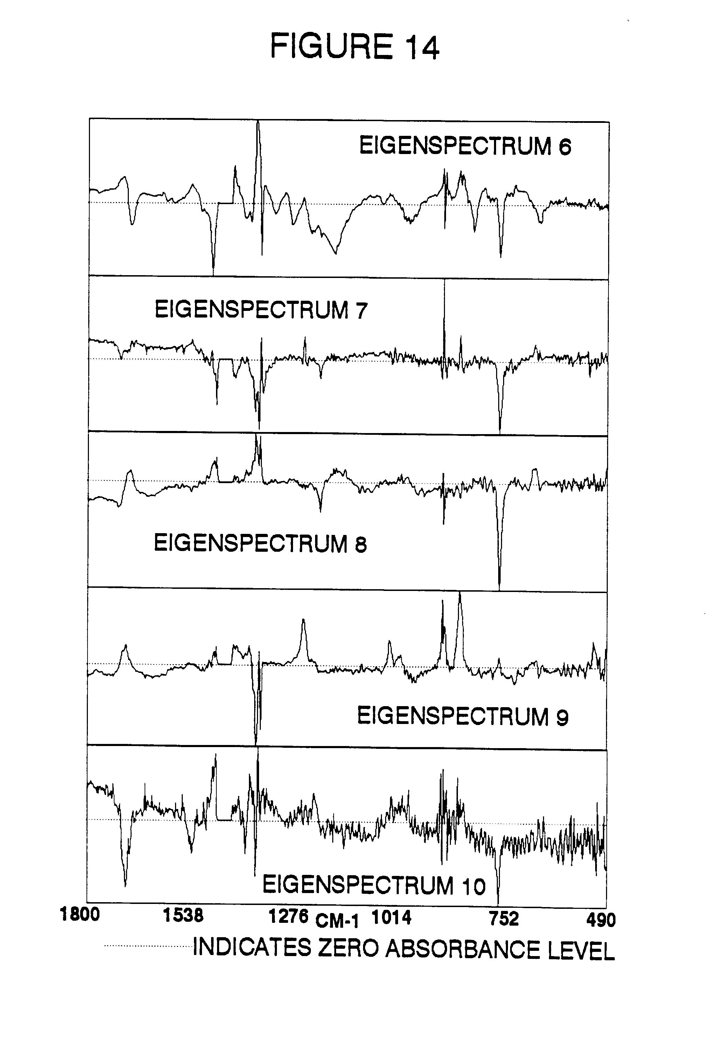

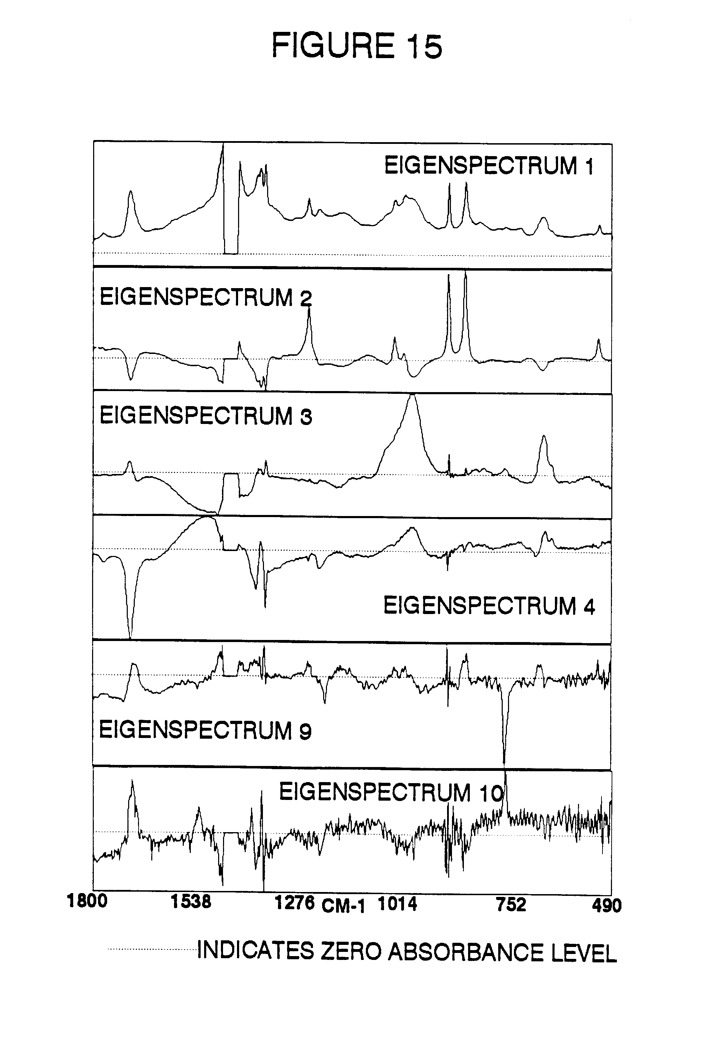

To evaluate what possible measurement process signals might be present in the spectral data of the calibration blends, a Principal Components Analysis was conducted on the 15 spectra. Figure 12 shows the statistics for the PCA calculation. The various statistical tests do not clearly indicate or agree on the number of variables present in the spectral data, although most of the tests suggest that there are more variables than real components. Figures 13 and 14 show the first 10 eigenspectra obtained for the PCA calculation. Clearly, at least the first 9 eigenspectra contain recognizable absorption bands which are well above the spectral noise level. Examination of the eigenspectra and the standard deviation spectrum indicate the sources of the additional spectral variables. Eigenspectra 8 shows negative bands at 1215 and 761 cm$^{-1}$ which are due to chloroform contamination of the solutions, chloroform having been used to rinse the cell between samples. The standard deviation spectrum (Figure 11) shows a strongly curved background, suggesting that background variations may also be contributing the spectral variance. Unlike previous examples, the contributions of the background to the spectral variables are not as obvious from the eigenspectra, but are mixed with the variations due to the real component absorptions. Together, the chloroform and the background could account for two of the nine spectral variables. Eigenspectra 5 and 9 both show features due to the cyclohexane solvent. In eigenspectrum 5, the bands have a dispersive shape suggestive of a variation in bandwidth among the calibration spectra. Close examination of the calibration spectra indicates that the cyclohexane absorptions do vary slightly in bandwidth, presumably because of variations in the strength of interactions with the additives. Eigenspectrum 9, on the other hand, shows normally shaped features corresponding to the solvent. If the solutions had all been prepared to exactly the same concentration, and if no solvent/additive interactions were present, the spectral features due to the cyclohexane would have been constant among all the blends, would have been observed exclusively in eigenspectrum 1, and would not have been detected as a separate spectral variable. In reality, the solvent contributes two spectral variables, eigenspectrum 5 arising from variations in the solute/solvent interactions, and eigenspectrum 8 arising from variations in the solute/solvent concentrations. To verify that this is the case, a second PCA analysis was conducted using the 15 calibration blends plus 3 spectra of cyclohexane obtained under the same conditions. Figure 15 shows some of the eigenspectra obtained from this analysis. In the PCA calculation using only the blend spectra, the variation in the solvent concentration was small, and showed up only as the $9^{th}$ most important spectral variable (Figure 14). Adding the spectra of the solvent to the reference set dramatically increases the range of variation in the solvent absorptions, making it the $2^{nd}$ most important spectral variable. Aside from the noise level, eigenspectrum 2 in Figure 15 very closely resembles the eigenspectrum 9 in Figure 14. If solvent concentration variation was not a spectral variable in the original 15 calibration spectra, then the inclusion of the solvent spectra would have added an additional variable to the data. Comparison of Figures 13 and 14 to Figure 15 clearly indicates that this is not the case. The inclusion of the solvent spectra has reordered the 9 variables (e.g. eigenspectrum 2 in Figure 13 becomes eigenspectrum 3 in Figure 14), but the 10th eigenspectrum in both cases shows minimal absorption features above the spectral noise level.

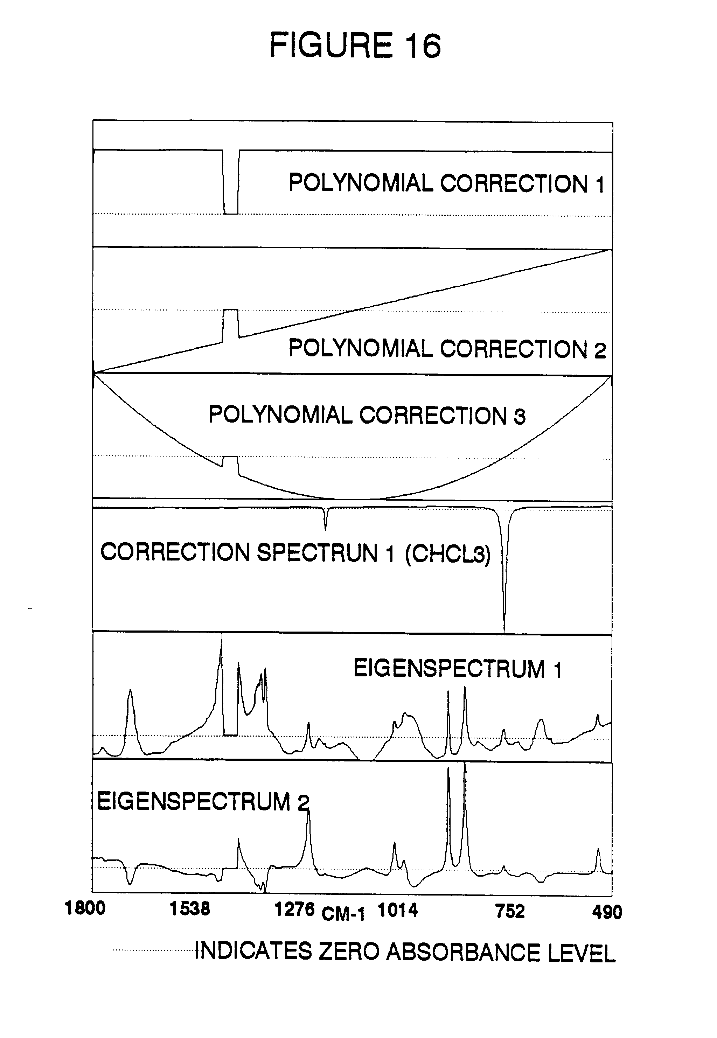

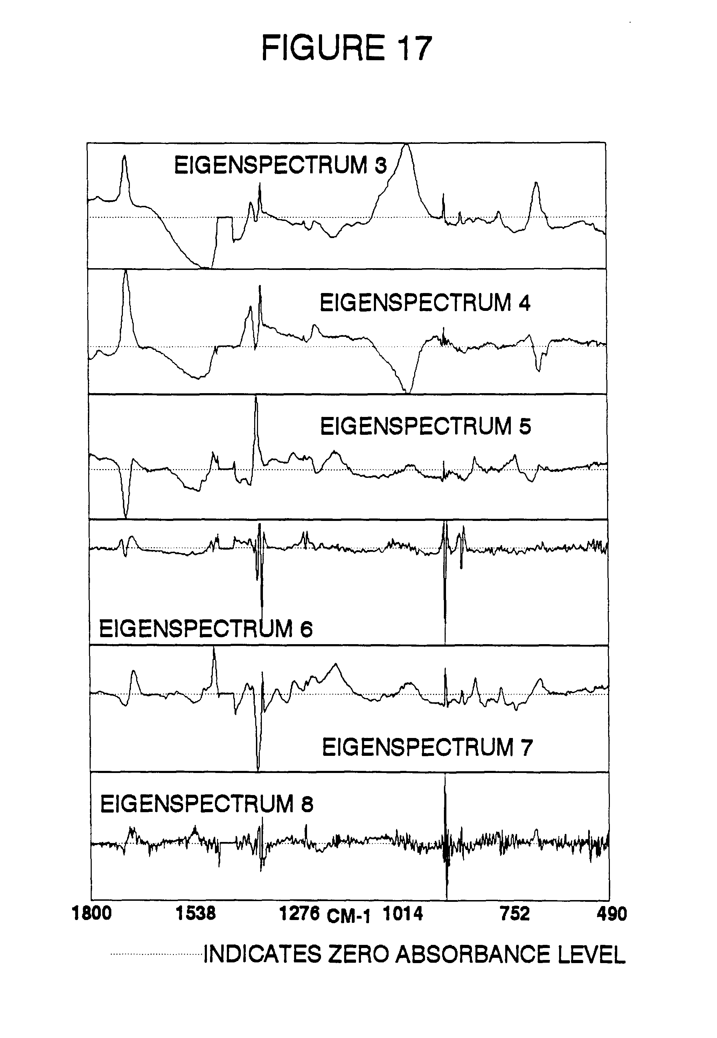

The above analysis indicates that 7 of the 9 spectral variables correspond to sample variations, 5 due to additive package components, 1 due to variations in the cyclohexane solvent concentration, and 1 due to variations in the solvent/solute interactions. The remaining 2 variables are due to the measurement process, and correspond to background fluctuations and chloroform contamination. Using this information, a CPSA model can be constructed. Since the solvent concentration variation is a variable in the spectra of the blends, the solvent spectra were included in the reference set for the generation of the CPSA model so as to ensure that the solvent variation could be properly modeled. Figures 16 and 17 show the various correction spectra which are used, as well as the resulting eigenspectra. A 3 term (quadratic) correction is used to account for the possible background variation. Although the chloroform is actually in the sample, it is at a low enough level so as not to cause significant dilution of the sample absorptions, and thus can be treated as a measurement process variable rather than a sample component. Since the chloroform absorptions observed in PCA eigenspectra 8 are shifted slightly from those observed for neat chloroform, a synthetic *chloroform* spectrum was generated for use as a correction spectrum by fitting the observed absorptions to Lorenztian bands. CPSA eigenspectrum 7 (Figure 16) appears to be roughly equivalent to PCA eigenspectrum 10 (Figures 14 and 15) in terms of the signal to noise of the residual absorption features. By including the 4 constraints, the CPSA model allows the spectral variance to be accounted for in 7 variables rather than the 9 required by the PCA analysis.

Table 3 shows the Standard Errors for the K Matrix, PCA and CPSA models. Since there are only 6 components (5 add pack components plus cyclohexane) in the samples, the K Matrix method can only extract 6 variables. The K Matrix model cannot account for the solute/solvent interactions and thus produces a poorer predictive model than the Principal Component methods which allow this interaction to be modeled as a separate variable. The Standard Error of Estimate is lower for the PCA model based on 9 variables than the CPSA model based on 7, because the two extra variable that correspond to measurement process signals are being correlated to the dependent variable. If, for instance, we use the PCA model to analyze the 4 constraint spectra used for the CPSA model (the first 4 spectra in Figure 16), we see (Table 4) that predictions made with the PCA model will depend on background and chloroform contamination, and will not be robust relative to these measurement process signals. When the PCA and CPSA models are used to analyze the 15 test blends (Table 3, bottom), it can be seen that the predictive capability of the more robust CPSA model based on 7 variables actually exceeds that of the PCA model based on 9 variables.

For simplicity, since no water vapor absorptions were observed in the standard deviation spectra or the PCA eigenspectra, a water vapor constraint was not added in the development of the above CPSA model. If this model was to be used in actual quality control applications, a water vapor constraint would be added to insure that the results were not affected by the instrument purge. As is seen in Table 3, the addition of water vapor correction spectra to the model so as to improve its stability and robustness does not effect the accuracy of the predictions for the 15 test spectra which showed no water vapor absorptions.

**Example 8:**

Example 8 is intended to demonstrate how a Constrained Spectral Analysis can be employed to remove a signal arising from the measurement process in a real application. A CPCR model was developed to estimate the research octane number (RON) of powerformate samples. Laboratory data was collected over the 870 to 1600 namometer range for 186 reference powerformate samples using a LTI Industries Quantum 1200 Near-Infrared Analyzer. RON values for the powerformate reference samples were obtained via ASTM-2699. A CPCR model was developed using 3 polynomial correction spectra (constant, linear and quadratic polynomials), and 5 Constrained Principal Component variables. The Standard Error of Estimate for the model was 0.30 research octane numbers.

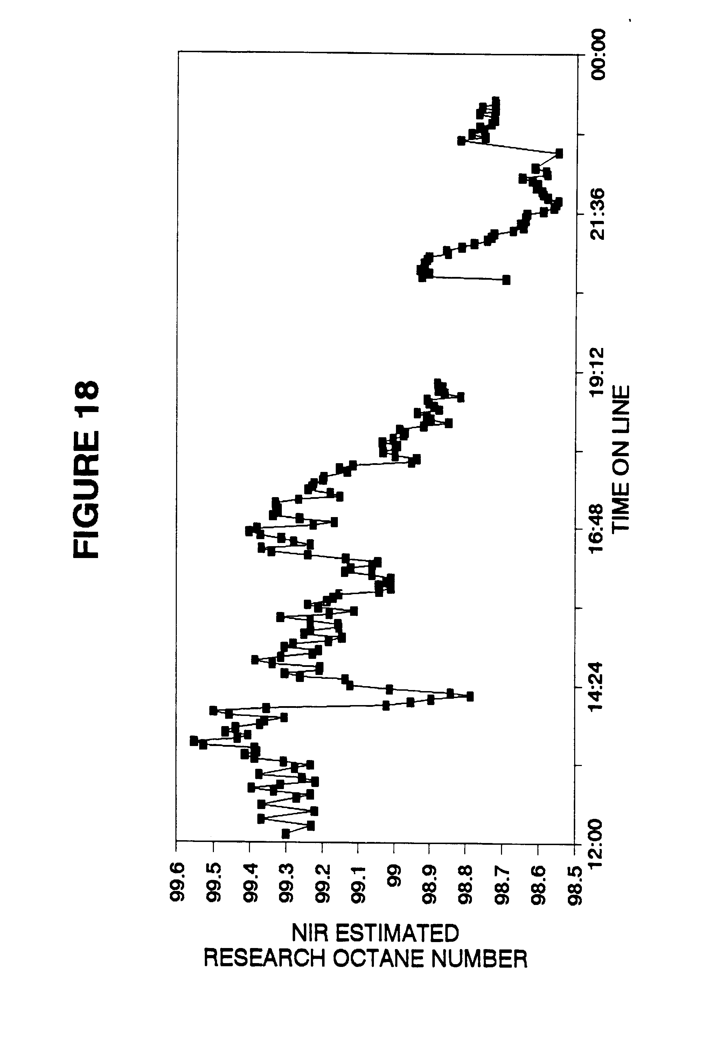

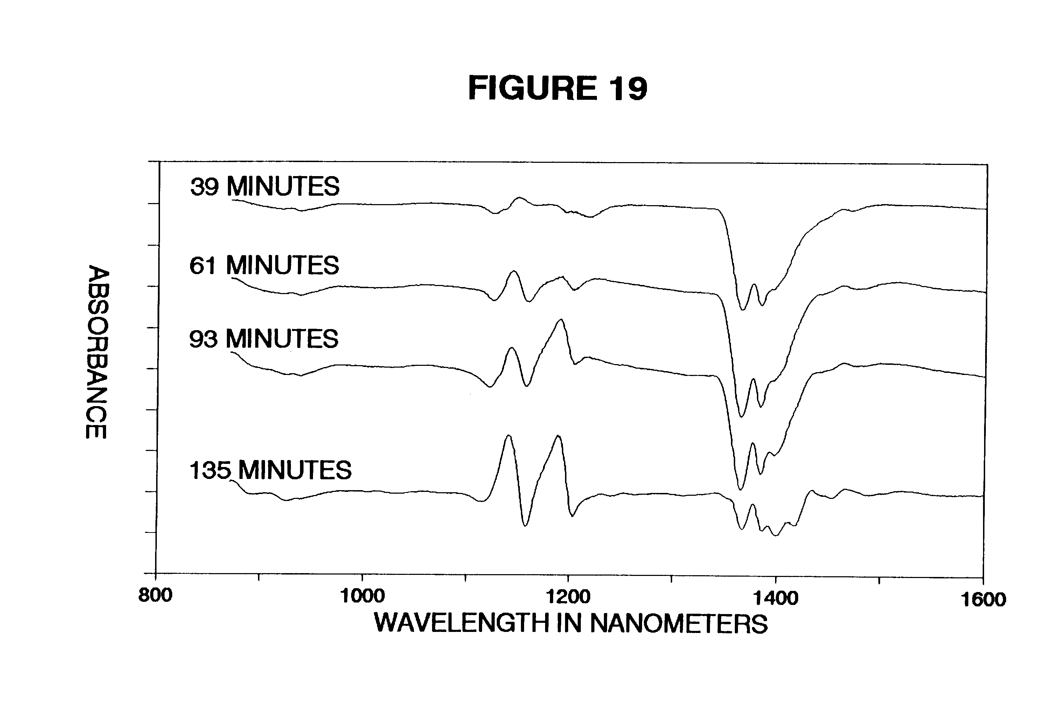

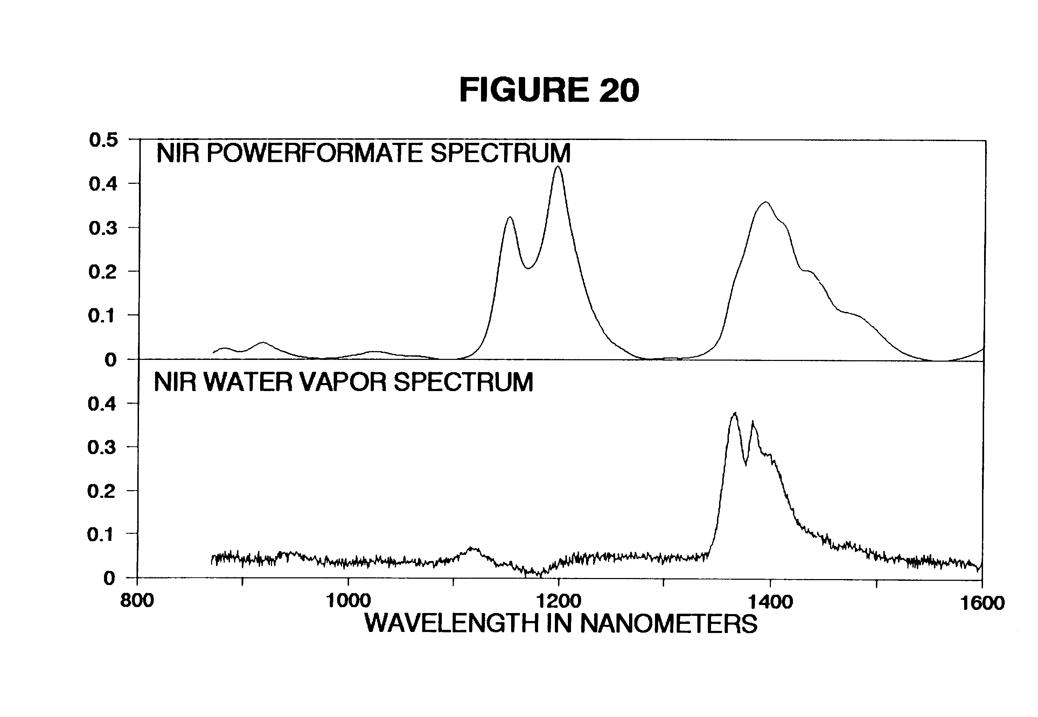

In testing the viability of using Near-Infrared for the measurement of on-line research octane number, the laboratory analyzer was equiped with a flow cell, and connected to a fast sampling loop from a powerforming unit. Spectra of the powerforming product were collected at approximately 6 minute intervals using the same conditions as were used for the collection of the reference spectra. The resultant estimate of the research octane number (Figure 18) showed a periodic oscillation at a frequency which was much more rapid than expected changes in the product composition. By subtracting successive spectra (Figure 19), it was established that the oscillation was due to a periodic variation in the absorption the range of 1400 nanometers. The absorption was identified as being due to atmospheric water vapor (Figure 20) which was present in the instrument light path, and the oscillation was tracked to the cycle time of an air conditioning unit present in the instrument shake where the analyzer was located. From Figure 19, it is clear that the for short time periods (<40 minutes), there were only minor changes in the powerformate product composition, and the periodic changes in the estimated RON where due soley to the variations in the humidity in the instrument. Difference spectra generated over longer time intervals (e.g. 93-135 minutes) demonstrated that the magnitude of the water vapor absorption was comparable to the differences in absorption due to actual compositional changes. From Figure 20, it can be seen that the water vapor absorption falls within the same spectral range as absorptions due to the powerformat hydrocarbons.

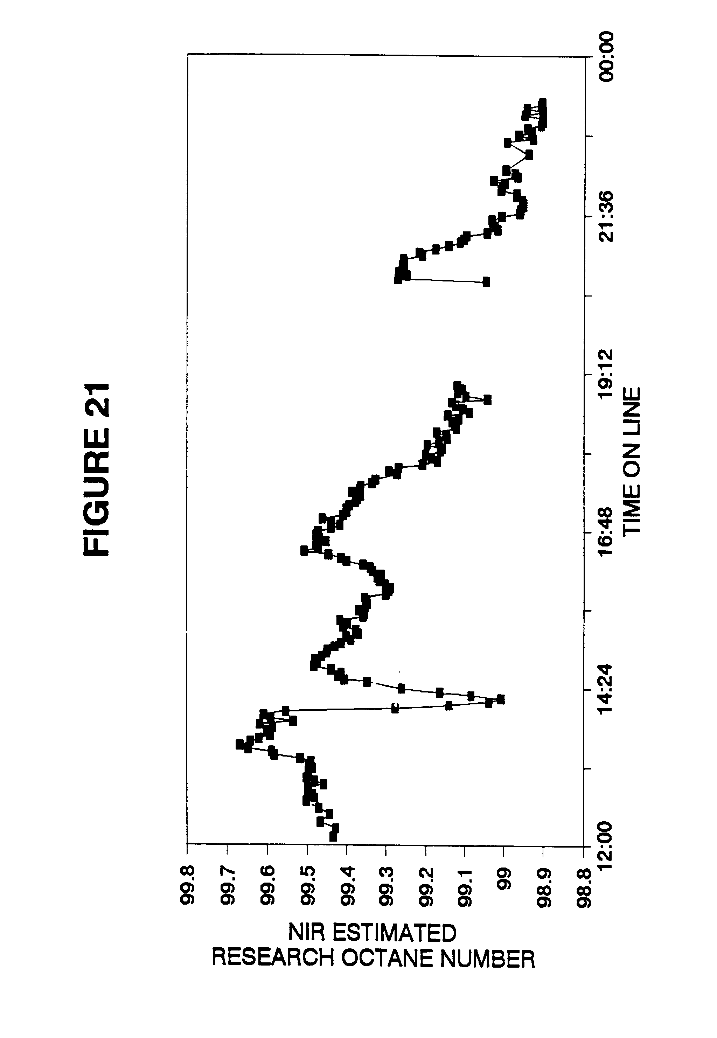

To minimize the effect of humidity variations on the estimation of research octane number, a water vapor correction was added to the model. A "water vapor" spectrum was generated by subtracting successive spectra from the on-line data over periods when the composition was expected to show minimal variation. A CPCR model was constructed using the 3 polynomial background corrections, the water vapor correction, and using 5 Constrained Principal Components in the regression. The resultant model was then used to reanalyze the same on-line data (Figure 21). The inclusion of the water vapor correction eliminated the periodic oscillation by producing a model that was insensitive to variations in the humidity.

### Example 9: Measurement Process Quality Control

CPSA allows the spectral variables which are associated with the measurement process to be modeled so that the predictive models can be constrained to be insensitive to these variables. The contribution of these constraint variables to any real spectrum represents the status of the measurement process during the collection of the spectral data. Since the constraint variables are orthogonal among themselves and to the eigenspectra, the relative contributions of the constraint variables to a spectrum are obtained merely by taking the dot product of the constraint vector with the spectrum. The range of values obtained for the calibration spectra represent the range of measurement process variation during the collection of the reference spectra, and can be used to identify reference spectra which are outliers with respect to the measurement process. In building the predictive model it may be desirable to recollect the spectral data for these outliers so as to optimize the model. Similarly, the values of the constraint variables for an unknown spectrum being analyzed serve as an indication of measurement process during the analysis, and can provide a warning if the measurement process has changed significantly relative to the calibration. CPSA thus provide a means of performing quality control on the measurement process, indicating conditions under which changes in the measurement process rather than changes in the sample might be affecting the predicted results.

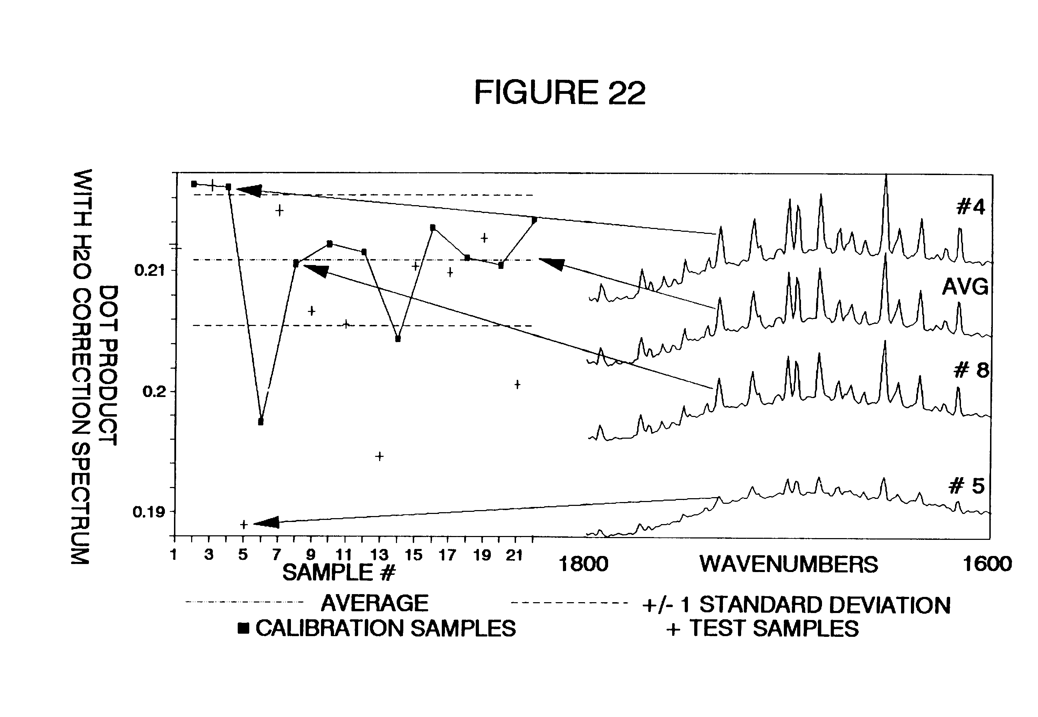

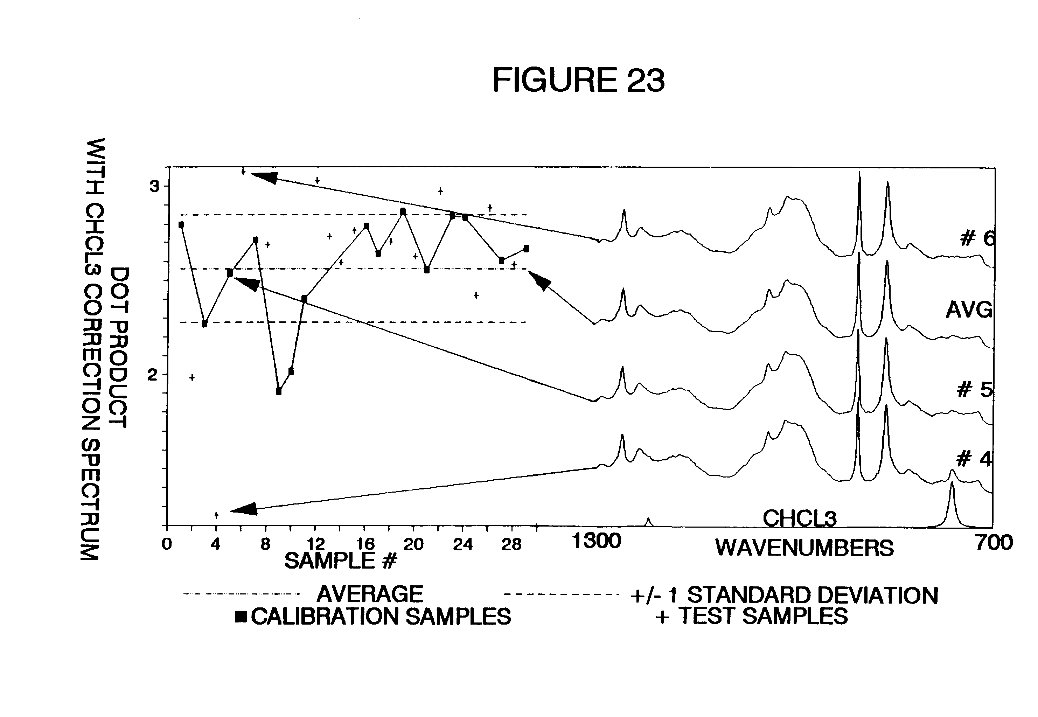

Figures 22 and 23 show examples of how the constraint variables can be used to monitor the spectral measurement process. In the isooctane/heptane example above (Example 6), the spectra were collected in a rapid fashion without allowing sufficient time for the spectrometer to be fully purged. As a result, absorptions due to water vapor are superimposed on the component spectra, and are isolated as a Principal Component (eigenspectrum 5 in Figure 8) by a PCA calculation. In developing the CPSA model, water vapor correction spectra were used to generate a constraint. Figure 18 shows a plot of the dot product of the reference and test spectra with the water vapor constraint. The value of the constraint variable clearly identifies spectra which are outliers with respect to the water vapor level, in this case spectra which show a significantly lower water vapor level than the average. Figure 23 shows similar data for the chloroform constraint used in the additive package example (Example 7). Chloroform was used to rinse the cell between samples, tends to penetrate into small openings between the spacer and the windows, and is not always completely removed even when a vacuum is pulled on the cell. The value of the chloroform constraint variable clearly indicates spectra for which an above (sample 4) or below (sample 6) average amount of chloroform contamination was present. Note that since the peaks in the chloroform constraint spectrum (Figure 16) are negative, lower values of the dot product imply higher levels of chloroform.

It will be appreciated that various modifications and changes may be made to the methods and apparatus disclosed herein within the scope of the invention as defined by the appended claims. Such modifications and changes will be obvious to the skilled addressee and will not be further described herein.

Table 1

| Binary Isooctane/Heptane Samples | | |
|---|---|---|
| | Isooctane Volume % | Heptane Volume % |
| Calibration Samples | | |
| 2 | 78 | 22 |
| 4 | 82 | 18 |
| 6 | 84 | 16 |
| 8 | 86 | 14 |
| 10 | 88 | 12 |
| 12 | 90 | 10 |
| 14 | 92 | 8 |
| 16 | 94 | 6 |
| 18 | 96 | 4 |
| 20 | 98 | 2 |
| 22 | 100 | 0 |
| Test Samples | | |
| 1 | 76 | 24 |
| 3 | 80 | 20 |
| 5 | 83 | 17 |
| 7 | 85 | 15 |
| 9 | 87 | 13 |
| 11 | 89 | 11 |
| 13 | 91 | 9 |
| 15 | 93 | 7 |
| 17 | 95 | 5 |
| 19 | 97 | 3 |
| 21 | 99 | 1 |

Table 2

| Standard Errors for Heptane/Isooctane Binary Mixtures | | | | | |
|---|---|---|---|---|---|
| Type of Model | # of PC | Isooctane | | Heptane | |
| | | SEE | SEP | SEE | SEP |
| Models based on 11 reference spectra without pathlength correction: | | | | | |
| PCR | 2 | 2.715 | 3.396 | 6.736 | 8.198 |
| | 3 | 0.263 | 0.551 | 0.466 | 1.081 |
| | 4 | 0.197 | 0.187 | 0.035 | 0.077 |
| | 5 | 0.118 | 0.213 | 0.035 | 0.085 |
| | 6 | 0.129 | 0.218 | 0.030 | 0.062 |
| CPCR | 2 | 0.196 | 0.248 | 0.045 | 0.056 |
| Results renormalized so heptane plus isooctane equals 100%: | | | | | |
| PCR | 4 | 0.040 | 0.077 | 0.040 | 0.077 |
| | 5 | 0.028 | 0.094 | 0.028 | 0.094 |

Table 2   (continued)

| Standard Errors for Heptane/Isooctane Binary Mixtures | | | | | |
|---|---|---|---|---|---|
| Type of Model | # of PC | Isooctane | | Heptane | |
| Results renormalized so heptane plus isooctane equals 100%: | | | | | |
| CPCR | 2 | 0.056 | 0.071 | 0.056 | 0.071 |
| Results for models employing pathlength correction: | | | | | |
| PCR | 2 | 6.394 | 6.986 | 6.394 | 6.986 |
| | 3 | 0.445 | 0.977 | 0.445 | 0.977 |
| | 4 | 0.045 | 0.072 | 0.045 | 0.072 |
| | 5 | 0.034 | 0.091 | 0.034 | 0.091 |
| CPCR | 2 | 0.056 | 0.071 | 0.056 | 0.071 |
| SEE = Standard Error of Estimate for Calibration | | | | | |
| SEP = Standard Error of Prediction for Test Set | | | | | |

Table 3

| Comparison of K Matrix, PCA and CPSA Results for Add Pack Analysis | | | | | | |
|---|---|---|---|---|---|---|
| Models based on 15 blend spectra plus cyclohexane | | | | | | |
| Results renormalized so 5 add pack components add to 100% | | | | | | |
| Model | # of Var. | Standard Error of Estimate | | | | |
| | | Disp | NPS | ZDDP | MgSulf | Oil |
| K Matrix | 6 | 0.369 | 0.363 | 0.150 | 0.164 | 0.704 |
| PCA | 9 | 0.148 | 0.217 | 0.106 | 0.088 | 0.126 |
| CPSA | 7 | 0.291 | 0.271 | 0.143 | 0.128 | 0.220 |
| CPSA * | 7 | 0.299 | 0.273 | 0.144 | 0.127 | 0.229 |
| Model | # of Var. | Standard Error of Prediction | | | | |
| | | Disp | NPS | ZDDP | MgSulf | Oil |
| K Matrix | 6 | 0.412 | 0.398 | 0.132 | 0.300 | 0.612 |
| PCA | 9 | 0.429 | 0.285 | 0.147 | 0.300 | 0.367 |
| CPSA | 7 | 0.410 | 0.267 | 0.098 | 0.314 | 0.378 |
| CPSA * | 7 | 0.402 | 0.263 | 0.099 | 0.313 | 0.375 |
| # of Var. = Number of variables in model | | | | | | |
| Disp = Dispersant, NPS = Nonyl Phenol, ZDDP = Zinc | | | | | | |
| Dialkyl-Dithiophosphate, MgSulf = Magnesium Sulfonate | | | | | | |

* CPSA model including water vapor constraint

Table 4

| PCA Analysis of CPSA Constraints | | | | | |
|---|---|---|---|---|---|
| Constraint | Disp | NPS | ZDDP | MgSulf | Oil |
| Polynomial 1 (constant) | | | | | |
| | 0.902 | 2.094 | 1.342 | 1.436 | 2.521 |
| Polynomial 2 (linear) | | | | | |
| | -5.500 | 2.099 | 1.149 | -0.843 | 12.110 |
| Polynomail 3 (square) | | | | | |
| | -1.321 | -5.889 | -1.147 | 1.009 | 1.060 |
| Correction 1 ($CHCL_3$) | | | | | |
| | -0.221 | -0.34 | 0.213 | -0.105 | 0.230 |

The Constrained Principal Spectra Analysis method allows the spectroscopist to input his knowledge of the spectral measurements so as to define and model possible spectral variations which are due to the measurement process, and to develop multivariate predictive models which are constrained to be insensitive to the measurement process signals. The constraints developed in this fashion serve as quality control variables for the measurement process, allowing for the optimization of the calibration and subsequent monitoring of the spectral measurement. The CPSA algorithm provides advantage over spectral preprocessing techniques in that: (1) it uses all available spectral data to derive and remove the measurement process variables and as such is less sensitive to spectral noise than methods based on limited ranges of the data, (2) it uses a single calculation method to remove all types of measurement process variations including variables for which preprocessing algorithms would be difficult or impossible to develop (e.g highly overlapping interferences or higher order background variations), (3) it provides measurement process variables which are orthogonal to the spectral Principal Components thereby insuring maximum stability of the predictive model, and (4) it incorporates the modeling and removal of measurement process variables as an integral part of the analysis thereby removing the necessity for the development of separate preprocessing methods and algorithms.

It will be appreciated that various modifications and changes may be made to the methods and apparatus disclosed herein within the scope of the invention as defined by the appended claims. Such modifications and changes will be obvious to the skilled addressee and will not be further described herein.

## Claims

1. A method of quantitatively estimating unknown property and/or composition data of a sample, comprising:

(i) collecting respective infrared spectra of n calibration samples, the spectra being quantified at $f$ discrete frequencies (or wavelengths) and forming a matrix $\mathbf{X}$ of dimension $f$ by $n$;

(ii) producing a correction matrix $\mathbf{U_m}$ of dimension $f$ by $m$ comprising $m$ digitised correction spectra at said discrete frequencies $f$, said correction spectra simulating data arising from the measurement process itself;

(iii) orthogonalising $\mathbf{X}$ with respect to $\mathbf{U_m}$ to produce a corrected spectra matrix $\mathbf{X_c}$ whose spectra are each orthogonal to all the spectra in $\mathbf{U_m}$;

(iv) collecting c property and/or composition data for each of the $n$ calibration samples to form a matrix $\mathbf{Y}$ of dimension $n$ by $c$ ($c > 1$);

(v) determining a predictive model correlating the elements of matrix $\mathbf{Y}$ to those of matrix $\mathbf{X_c}$;

(vi) measuring the infrared spectrum of the sample under consideration at said $f$ discrete frequencies to form a matrix of dimension $f$ by 1; and

(vii) quantitatively estimating the unknown property and/or composition data of the sample under consideration from its measured spectrum using the predictive model.

2. A method according to claim 1, wherein, for determining any significant change in the measurement process data between the times steps (i) and (vi) were performed, the following steps are carried out:

(a) a matrix $\mathbf{V_m}$ of dimension $n$ by $m$ is formed as the dot product $\mathbf{X^tU_m}$, where $\mathbf{X^t}$ is the transpose of matrix $\mathbf{X}$;

(b) the corrected data matrix $\mathbf{X_c}$ is formed and its singular value decomposition computed as $\mathbf{U\Sigma V^t}$, where $\mathbf{U}$ is a matrix of dimension $f$ by $n$ and contains the principal component spectra as columns, $\Sigma$ is a diagonal matrix of dimension $n$ by $n$ and contains the singular values, and $\mathbf{V}$ is a matrix of dimension $n$ by $n$ and contains the principal component scores, $\mathbf{V^t}$ being the transpose of $\mathbf{V}$;

(c) a regression of the form $\mathbf{V_m = VZ + R}$ is determined;

(d) a vector $\mathbf{v_m}$ is formed as the dot product of the measured spectrum of the sample under consideration, $\mathbf{x_u}$, with each of the columns of the correction matrix $\mathbf{U_m}$, $\mathbf{v_m = x^t_uU_m}$;

(e) a corrected spectrum $\mathbf{x_c}$ is formed as a result of orthogonalising $\mathbf{x_u}$ with respect to $\mathbf{U_m}$, $\mathbf{x_c = x_u - U_mv^t_m}$;

(f) the scores for the corrected spectrum are calculated as $\mathbf{v = x_c^t U\Sigma^{-1}}$ where $\mathbf{x_c^t}$ is the transpose of $\mathbf{x_c}$;

(g) the measurement process signals are calculated as $\mathbf{r = v_m - vZ}$; and

(h) the magnitude of the elements of r are compared with the range of values of $\mathbf{R}$, whereby a significant difference indicates a significant change in the measurement process data.

3. A method as claimed in claim 1 or claim 2, wherein the predictive model is determined in step (v) using a mathematical technique to solve the equation $\mathbf{Y = X_c^t P + E}$, where $\mathbf{X_c^t}$ is the transpose of $\mathbf{X_c}$, $\mathbf{P}$ is a prediction matrix of dimension $f$ by $c$, and $\mathbf{E}$ is a matrix of residual errors from the model and is of dimension $n$ by $c$ and wherein, for determining the vector $\mathbf{y_u}$ of dimension 1 by $c$ containing the estimates of the $c$ property and/or composition data for the sample under consideration, the spectrum $\mathbf{x_u}$ of the sample under consideration, $\mathbf{x_u}$ being of dimension $f$ by 1, is measured and $\mathbf{y_u}$ is determined from the relationship $\mathbf{y_u = x_u^t P}$, $\mathbf{x_u^t}$ being the transpose of spectrum vector $\mathbf{x_u}$.

4. A method as claimed in claim 3, wherein said mathematical technique is principal components regression.

5. A method as claimed in any one of the preceding claims, wherein the $m$ spectra forming the columns of matrix $\mathbf{U_m}$ are all mutually orthogonal.

6. A method as claimed in any one of claims 1 to 4, wherein step (ii) comprises:-

(iia) modelling baseline variations by a set of orthonormal frequency (or wavelength) dependent polynomials which form the columns of a matrix $\mathbf{U_p}$ of dimension $f$ by $p$ where $p$ is the order of the polynomials and the columns of $\mathbf{U_p}$ are orthonormal polynomials;

(iib) supplying at least one ex-sample spectrum which is representative of an anticipated spectral interference due to an ex-sample chemical compound, this ex-sample spectrum or these ex-sample spectra forming the column(s) of a matrix $\mathbf{X_s}$ of dimension $f\ by\ s$ where $s$ (> 1) is the number of such ex-sample spectra and is greater than or equal to the number of such ex-sample spectral interferences $s'$;

(iic) orthogonalising the column(s) of $\mathbf{X_s}$ with respect to $\mathbf{U_p}$ to form a new matrix $\mathbf{X_s}'$;

(iid) orthogonalising the column(s) of $\mathbf{X_s}'$ among themselves to form a new matrix $\mathbf{U_s}$;

(iie) combining matrices $\mathbf{U_p}$ and $\mathbf{U_s}$ to form a matrix $\mathbf{U_m}$ whose columns are the columns of $\mathbf{U_p}$ and $\mathbf{U_s}$ arranged side-by-side.

7. Apparatus for quantitatively estimating unknown property and/or compositional data of a sample, comprising :-

- a spectrometer for generating the respective infrared spectrum of a plurality of calibration samples $n$ having known properties and/or composition $c$, and the infrared spectrum of a sample under consideration having unknown property and/or composition data which is to be estimated; and

- computer means arranged to receive the measured spectrum data from the spectrometer and operable

(i) in a data correction mode to perform, in conjunction with an operator, steps (i) to (iii) of claim 1;

(ii) in a storing mode to store the $c$ property and/or compositional data for each of the $n$ calibration samples to form the matrix **Y** of dimension $n$ by $c$ $(c \geq 1)$;

(iii) in a model building mode to determine, in conjunction with the operator, a predictive model according to step (v) of claim 1;

(iv) in a measurement mode to perform step (vi) of claim 1; and

(v) in a prediction mode to perform step (vii) of claim 1, in order to quantitatively estimate the unknown property and/or composition data of the sample under consideration according to the determined predictive model correlating the elements of matrix **Y** to the corrected spectra matrix $\mathbf{X_c}$ resulting from the orthogonalization of matrix **X** with respect to the correction matrix $\mathbf{U_m}$.

## Patentansprüche

1. Verfahren zum quantitativen Abschätzen unbekannter Eigenschafts- und/oder Zusammensetzungsdaten einer Probe, mit:

(i) Aufnehmen jeweiliger Infrarotspektren von $n$ Kalibrierungsproben, wobei die Spektren bei $f$ diskreten Frequenzen (oder Wellenlängen) quantifiziert sind und eine Matrix **X** der Dimension $f$ mal $n$ bilden;

(ii) Erzeugen einer Korrekturmatrix $\mathbf{U_m}$ der Dimension $f$ mal $m$ mit $m$ digitalisierten Korrekturspektren bei den diskreten Frequenzen $f,$ wobei die Korrekturspektren aus dem Meßprozeß selbst entstehende Daten simulieren;

(iii) Orthogonalisieren von **X** in bezug auf $\mathbf{U_m}$, um eine korrigierte Spektrenmatrix $\mathbf{X_c}$ zu erzeugen, deren Spektren jeweils orthogonal zu allen Spektren in $\mathbf{U_m}$ sind;

(iv) Sammeln von c Eigenschafts- und/oder Zusammensetzungsdaten für jede der $n$ Kalibrierungsproben, um eine Matrix **Y** der Dimension $n$ mal $c$ $(c > 1)$ zu bilden;

(v) Bestimmen eines Vorhersagemodells, das die Elemente der Matrix **Y** mit denen der Matrix $\mathbf{X_c}$ korreliert;

(vi) Messen des Infrarotspektrums der betrachteten Probe bei den $f$ diskreten Frequenzen, um eine Matrix der Dimension $f$ mal 1 zu bilden; und

(vii) quantitatives Abschätzen der unbekannten Eigenschafts- und/oder Zusammensetzungsdaten der betrachteten Probe aus ihrem gemessenen Spektrum unter Verwendung des Vorhersagemodells.

2. Verfahren nach Anspruch 1, wobei die folgenden Schritte ausgeführt werden, um irgendwelche signifikanten Änderungen in den Meßprozeßdaten zwischen den Zeiten, wenn die Schritte (i) und (vi) ausgeführt wurden, zu bestimmen:

(a) eine Matrix $\mathbf{V_m}$ der Dimension $n$ mal $m$ wird als inneres Produkt $\mathbf{X^t U_m}$ gebildet, wobei $\mathbf{X^t}$ die Transponierte der Matrix **X** ist;

(b) die korrigierte Datenmatrix $\mathbf{X_c}$ wird gebildet und ihre Singularwertzerlegung als $\mathbf{U\Sigma V^t}$ berechnet, wobei **U** eine Matrix der Dimension $f$ mal $n$ ist und die Hauptkomponentenspektren als Spalten enthält, $\Sigma$ eine Diagonalmatrix der Dimension $n$ mal $n$ ist und die Singularwerte enthält und **V** eine Matrix der Dimension $n$ mal $n$ ist und die Hauptkomponentenwertungen enthält, wobei $\mathbf{V^t}$ die Transponierte von **V** ist;

(c) eine Regression der Form $V_m = VZ + R$ wird bestimmt;

(d) ein Vektor $v_m$ wird als das innere Produkt des gemessenen Spektrums der betrachteten Probe, $x_u$, mit jeder der Spalten der Korrekturmatrix $U_m$ gebildet, $v_m = x_u^t U_m$;

(e) ein korrigiertes Spektrum $x_c$ wird als Ergebnis einer Orthogonalisierung von $x_u$ in bezug auf $U_m$ gebildet, $x_c = x_u - U_m v_m^t$;

(f) die Wertungen für das korrigierte Spektrum werden als $v = x_c^t U \Sigma^{-1}$ berechnet, wobei $x_c^t$ die Transponierte von $x_c$ ist;

(g) die Meßprozeßsignale werden als $r = v_m - vZ$ berechnet; und

(h) die Größe der Elemente von $r$ wird mit dem Bereich der Werte von $R$ verglichen, wodurch eine signifikante Differenz eine signifikante Änderung in den Meßprozeßdaten anzeigt.

3. Verfahren nach Anspruch 1 oder 2, wobei das Vorhersagemodell in Schritt (v) unter Verwendung einer mathematischen Technik, um die Gleichung $Y = X_c^t P + E$ zu lösen, bestimmt wird, wobei $X_c^t$ die Transponierte von $X_c$ ist, $P$ eine Vorhersagematrix der Dimension $f$ mal $c$ und $E$ eine Matrix von Restfehlern von dem Modell ist und die Dimension $n$ mal $c$ hat und wobei zum Bestimmen des Vektors $y_u$ der Dimension 1 mal $c$, der die Abschätzungen der $c$ Eigenschafts- und/oder Zusammensetzungsdaten der betrachteten Probe enthält, das Spektrum $x_u$ der betrachteten Probe, wobei $x_u$ die Dimension $f$ mal 1 hat, gemessen wird und $y_u$ aus der Beziehung $y_u = x_u^t P$ bestimmt wird, wobei $x_u^t$ die Transponierte des Spektrumvektors $x_u$ ist.

4. Verfahren nach Anspruch 3, wobei die mathematische Technik eine Hauptkomponentenregression ist.

5. Verfahren nach einem der vorhergehenden Ansprüche, wobei die m Spektren, die die Spalten der Matrix $U_m$ bilden, alle wechselseitig orthogonal sind.

6. Verfahren nach einem der Ansprüche 1 bis 4, wobei der Schritt (ii) aufweist:

(iia) Modellieren von Basislinienvariationen durch einen Satz orthonormaler frequenzabhängiger (oder wellenlängenabhängiger) Polynome, die die Spalten einer Matrix $U_p$ der Dimension $f$ mal $p$ bilden, wobei $p$ der Grad der Polynome ist und die Spalten von $U_p$ orthonormale Polynome sind;

(iib) Liefern wenigstens eines Spektrums außerhalb der Probe, das repräsentativ für eine erwartete spektrale Interferenz infolge einer chemischen Verbindung außerhalb der Probe ist, wobei dieses Spektrum außerhalb der Probe oder diese Spektren außerhalb der Probe die Spalte(n) einer Matrix $X_s$ der Dimension $f$ mal $s$ bilden, wobei $s$ (> 1) die Zahl solcher Spektren außerhalb der Probe ist und größer ist als oder gleich ist zu der Zahl derartiger spektraler Interferenzen $s'$ außerhalb der Probe;

(iic) Orthogonalisieren der Spalte(n) von $X_s$ in bezug auf $U_p$, um eine neue Matrix $X_s$, zu bilden;

(iid) Orthogonalisieren der Spalte(n) von $X_s$, untereinander, um eine neue Matrix $U_s$ zu bilden;

(iie) Kombinieren der Matrizen $U_p$ und $U_s$, um eine Matrix $U_m$ zu bilden, deren Spalten die nebeneinander angeordneten Spalten von $U_p$ und $U_s$ sind.

7. Vorrichtung zum quantitativen Abschätzen unbekannter Eigenschafts- und/oder Zusammensetzungsdaten einer Probe, mit:

- einem Spektrometer zum Erzeugen des jeweiligen Infrarotspektrums einer Anzahl von Kalibrierungsproben $n$, die bekannte Eigenschaften und/oder Zusammensetzung $c$ haben, und wobei das Infrarotspektrum einer betrachteten Probe unbekannte Eigenschafts- und/oder Zusammensetzungsdaten hat, die abgeschätzt werden sollen; und

- einer Computereinrichtung, die dazu eingerichtet ist, die gemessenen Spektrumdaten von dem Spektrometer zu empfangen und betriebsfähig ist,

EP 0 552 300 B1

(i) in einer Datenkorrektur-Betriebsart in Verbindung mit einem Operateur die Schritte (i) bis (iii) aus Anspruch 1 durchzuführen;

(ii) in einer Speicher-Betriebsart die c Eigenschafts- und/oder Zusammensetzungsdaten für jede der $n$ Kalibrierungsproben zu speichern, um die Matrix **Y** der Dimension $n$ mal $c$ ($c \geq 1$) zu bilden;

(iii) in einer Modellbildungs-Betriebsart in Verbindung mit einem Operateur ein Vorhersagemodell gemäß dem Schritt (v) aus Anspruch 1 zu bestimmen;

(iv) in einer Messungs-Betriebsart den Schritt (vi) aus Anspruch 1 durchzuführen; und

(v) in einer Vorhersage-Betriebsart den Schritt (vii) aus Anspruch 1 durchzuführen, um die unbekannten Eigenschafts- und/oder Zusammensetzungsdaten der betrachteten Probe gemäß dem bestimmten Vorhersagemodell quantitativ abzuschätzen, das die Elemente der Matrix **Y** mit der korrigierten Spektrenmatrix **X$_c$** korreliert, die von der Orthogonalisierung der Matrix **X** in bezug auf die Korrekturmatrix **U$_m$** herrührt.

## Revendications

1. Procédé d'évaluation quantitative de données inconnues de propriété et/ou de composition d'un échantillon. comprenant les étapes consistant:

    (i) à recueillir des spectres infrarouges respectifs de n échantillons d'étalonnage, les spectres étant quantifiés à f fréquences (ou longueurs d'onde) discrètes et formant une matrice X de dimension f par n,

    (ii) à produire une matrice de correction $U_m$ de dimension f par m, comprenant m spectres de correction numérisés auxdites f fréquences discrètes, lesdits spectres de correction simulant des données provenant du procédé de mesure lui-même,

    (iii) à orthogonaliser X par rapport à $U_m$ pour produire une matrice de spectres corrigée $X_C$, dont les spectres sont individuellement orthogonaux à tous les spectres de $U_m$,

    (iv) à recueillir c données de propriété et/ou de composition pour chacun des n échantillons d'étalonnage pour former une matrice Y de dimension n par c (c>1),

    (v) à déterminer un modèle prédictif mettant en corrélation les éléments de la matrice Y avec ceux de la matrice $X_C$,

    (vi) à mesurer le spectre infrarouge de l'échantillon considéré auxdites f fréquences discrètes pour former une matrice de dimension f par 1, et

    (vii) à évaluer quantitativement les données inconnues de propriété et/ou de composition de l'échantillon considéré à partir de son spectre mesuré en utilisant le modèle prédictif.

2. Procédé selon la revendication 1, dans lequel, pour déterminer toute variation importante dans les données du procédé de mesure entre les moments où les étapes (i) et (vi) ont été effectuées, on procède aux étapes suivantes :

    (a) une matrice $V_m$ de dimension n par m est formée comme produit scalaire $X^t U_m$, où $X^t$ est la transposée de la matrice X,
    (b) la matrice de données corrigée $X_c$ est formée et sa décomposition en valeurs singulières calculée par $U\Sigma V^t$, où U est une matrice de dimension f par n et contient les spectres composants principaux en colonnes, $\Sigma$ est une matrice diagonale de dimension n par n et contient les valeurs singulières et V est une matrice de dimension n par n et contient les résultats composants principaux, $V^t$ étant la transposée de V.
    (c) on détermine une régression de forme $V_m = VZ + R$,
    (d) un vecteur $v_m$ est formé en tant que produit scalaire du spectre mesuré de l'échantillon considéré, $x_u$, avec chacune des colonnes de la matrice de correction $U_m$, $v_m = x^t_u U_m$,
    (c) un spectre corrigé $x_c$ est formé par orthogonalisation de $x_u$ par rapport à $U_m$, $x_c = x_u - U_m v^t_m$,
    (l) les résultats du spectre corrigé sont calculés par $v = x^t_c U\Sigma^{-1}$, où $x^t_c$ est la transposée de $x_c$.

(g) les signaux du procédé de mesure sont calculés par $r = v_m - vZ$, et

(h) la grandeur des éléments de r est comparée à la plage de valeurs de R, une différence notable indiquant une variation importante des données du procédé de mesure.

3. Procédé selon la revendication 1 ou 2, dans lequel le modèle prédictif est déterminé dans l'étape (v) en utilisant une technique mathématique pour résoudre l'équation $Y = X^t_c P + E$, où $X^t_c$ est la transposée de $X_c$, P est une matrice de prédiction de dimension f par c, et E est une matrice d'erreurs résiduelles du modèle et a des dimension de n par c, et dans lequel, pour déterminer le vecteur $y_u$ de dimension 1 par c contenant les évaluations des c données de propriété et/ou de composition pour l'échantillon considéré. le spectre $x_u$ de l'échantillon considéré, $x_u$ étant de dimension f par 1, est mesuré et $y_u$ est déterminé à partir de la relation $y_u = x^t_u P$, $x^t_u$ étant la transposée du vecteur spectral $x_u$.

4. Procédé selon la revendication 3, dans lequel ladite technique mathématique est une régression des composants principaux.

5. Procédé selon l'une quelconque des revendications précédentes. dans lequel les m spectres formant les colonnes de la matrice $U_m$ sont tous mutuellement orthogonaux.

6. Procédé selon l'une quelconque des revendications 1 à 4, dans lequel l'étape (ii) comprend :

(iia) la modélisation de variations de la ligne de base par un ensemble de polynômes orthonormaux qui sont fonction de la fréquence (ou de la longueur d'onde) et qui forment les colonnes d'une matrice $U_p$ de dimension f par p, où p est l'ordre des polynômes et les colonnes de Up sont des polynômes orthonormaux.

(iib) la fourniture d'au moins un spectre de l'ex-échantillon qui est représentatif d'une interférence spectrale anticipée due à un composé chimique de l'ex-échantillon, ce spectre de l'ex-échantillon ou ces spectres de l'ex-échantillon formant la ou les colonnes d'une matrice $X_S$ de dimension f par s, où s (>1) est le nombre de spectres de l'ex-échantillon et est supérieur ou égal au nombre de ces interférences spectrales s' de l'ex-échantillon,

(iic) l'orthogonalisation de la ou des colonnes de $X_s$ par rapport à $U_p$ pour former une nouvelle matrice $X_s'$,

(iid) l'orthogonalisation de la ou des colonnes de $X_s'$ entre elles pour former une nouvelle matrice $U_s'$, et

(iie) la combinaison des matrices $U_p$ et $U_s$ pour former une matrice $U_m$ dont les colonnes sont les colonnes de $U_p$ et $U_s$ agencées côte à côte.

7. Appareil d'évaluation quantitative de données inconnues de propriété et/ou de composition d'un échantillon, comprenant :

- un spectromètre pour générer le spectre infrarouge respectif d'une série de n échantillons d'étalonnage ayant des propriétés et/ou une composition connues c, et le spectre infrarouge d'un échantillon considéré ayant des données inconnues de propriété et/ou de composition qui doivent être évaluées. et

- des moyens informatiques agencés pour recevoir les données spectrales mesurées du spectromètre et destinés à fonctionner

(i) dans un mode de correction de données pour effectuer, conjointement avec un opérateur, les étapes (i) à (iii) selon la revendication 1,

(ii) dans un mode de stockage pour stocker les c données de propriété et/ou de composition pour chacun des n échantillons d'étalonnage pour former la matrice Y de dimension n par c (c≥1),

(iii) dans un mode de construction de modèle pour déterminer, conjointement avec l'opérateur, un modèle prédictif selon l'étape (v) de la revendication 1,

(iv) dans un mode de mesure pour effectuer l'étape (vi) de la revendication 1, et

(v) dans un mode de prédiction pour effectuer l'étape (vii) de la revendication 1, pour évaluer quantitativement les données inconnues de propriété et/ou de composition de l'échantillon considéré selon le modèle prédictif déterminé en mettant en corrélation les éléments de la matrice Y avec la matrice de spectres corrigée $X_C$ résultant de l'orthogonalisation de la matrice X par rapport à la matrice de correction $U_m$.

**FIGURE 1**

BENZENE WT% BY GAS CHROMATOGRAPHY

BENZENE WT% BY INFRARED

FIGURE 2

# FIGURE 3

**FIGURE 4**

# FIGURE 5

WEIGHT PERCENT ZDDP BY MID-IR

WEIGHT PERCENT ZDDP AS PREPARED

EP 0 552 300 B1

## FIGURE 6A

LOG
EIGNVALUE

PCA

CPSA

# OF PRINCIPAL COMPONENTS

## FIGURE 6B

INDICATOR
FUNCTION

PCA

CPSA

# OF PRINCIPAL COMPONENTS

## FIGURE 6C

CUMULATIVE
PERCENT
VARIANCE

PCA

CPSA

# OF PRINCIPAL COMPONENTS

## FIGURE 6D

EIGENVALUE
RATIO

CPSA

PCA

# OF PRINCIPAL COMPONENTS

# FIGURE 7

# FIGURE 8

EIGENSPECTRUM 1

EIGENSPECTRUM 2

EIGENSPECTRUM 3

EIGENSPECTRUM 4

EIGENSPECTRUM 5

EIGENSPECTRUM 6

2000                    CM-1                    690

········ INDICATES ZERO ABSORBANCE LEVEL

# FIGURE 9

CORRECTION
SPECTRUM 1
CONSTANT

CORRECTION
SPECTRUM 2
LINEAR

CORRECTION
SPECTRUM 3
WATER VAPOR

EIGENSPECTRUM 1

EIGENSPECTRUM 2

EIGENSPECTRUM 3

2000                    CM-1                    690

········ INDICATES ZERO ABSORBANCE LEVEL

# FIGURE 10

PREDICTED ISOOCTANE CONTENT (VOLUME %)

89.1

89

ACTUAL ISOOCTANE CONTENT

88.9

-5    -3    -1    1    3    5

ADDED LINEAR BACKGROUND
(MULTIPLES OF LINEAR BACKGROUND FOR STANDARD DEVIATION SPECTRUM)

■ PCA MODEL (4 PC)          * CPSA MODEL (2 PC + 3 CONSTRAINTS)

EP 0 552 300 B1

# FIGURE 11

AVERAGE SPECTRUM

STANDARD
DEVIATION
SPECTRUM
TIMES 20

3800          3120          2440     CM-1     1761          1081          401

EP 0 552 300 B1

## FIGURE 12A

LOG
EIGN VALUE

# OF PRINCIPAL COMPONENTS

## FIGURE 12B

INDICATOR
FUNCTION

# OF PRINCIPAL COMPONENTS

## FIGURE 12C

CUMULATIVE
PERCENT
VARIANCE

# OF PRINCIPAL COMPONENTS

## FIGURE 12D

EIGENVALUE
RATIO

# OF PRINCIPAL COMPONENTS

■ A - PCA (NO CC6)          ◇ B - PSA (CC6)          △ C - CPSA (CC6)

# FIGURE 13

EIGENSPECTRUM 1

EIGENSPECTRUM 2

EIGENSPECTRUM 3

EIGENSPECTRUM 4

EIGENSPECTRUM 5

1800    1538    1276 CM-1 1014    752    490

------ INDICATES ZERO ABSORBANCE LEVEL

# FIGURE 14

EIGENSPECTRUM 6

EIGENSPECTRUM 7

EIGENSPECTRUM 8

EIGENSPECTRUM 9

EIGENSPECTRUM 10

1800   1538   1276 CM-1 1014   752   490

············· INDICATES ZERO ABSORBANCE LEVEL

# FIGURE 15

EIGENSPECTRUM 1

EIGENSPECTRUM 2

EIGENSPECTRUM 3

EIGENSPECTRUM 4

EIGENSPECTRUM 9

EIGENSPECTRUM 10

1800   1538   1276 CM-1 1014   752   490

·············· INDICATES ZERO ABSORBANCE LEVEL

45

# FIGURE 16

POLYNOMIAL CORRECTION 1

POLYNOMIAL CORRECTION 2

POLYNOMIAL CORRECTION 3

CORRECTION SPECTRUN 1 (CHCL3)

EIGENSPECTRUM 1

EIGENSPECTRUM 2

1800     1538     1276 CM-1 1014     752     490

·········· INDICATES ZERO ABSORBANCE LEVEL

46

# FIGURE 17

INDICATES ZERO ABSORBANCE LEVEL

FIGURE 18

# FIGURE 19

ABSORBANCE

39 MINUTES

61 MINUTES

93 MINUTES

135 MINUTES

800      1000      1200      1400      1600

WAVELENGTH IN NANOMETERS

EP 0 552 300 B1

# FIGURE 20

NIR POWERFORMATE SPECTRUM

NIR WATER VAPOR SPECTRUM

WAVELENGTH IN NANOMETERS

EP 0 552 300 B1

FIGURE 21

FIGURE 22

## FIGURE 23

EP 0 552 300 B1